# EUROPÄISCHE PATENTSCHRIFT

(11) **EP 1 073 638 B1**
(45) Veröffentlichungstag und Bekanntmachung des Hinweises auf die Patenterteilung: **23.07.2008**
(21) Anmeldenummer: 99922102.1
(22) Anmeldetag: 19.04.1999
(51) Int. Cl.: C07D 213/82, C07D 239/42, C07D 277/56, C07D 233/90, A61K 31/44, A61K 31/505

(54) **HETEROZYKLISCHE SUBSTITUIERTE AMIDE, DEREN HERSTELLUNG UND ANWENDUNG**
HETEROCYCLICALLY SUBSTITUTED AMIDES, THEIR PRODUCTION AND THEIR USE
AMIDES SUBSTITUES PAR VOIE HETEROCYCLIQUE, LEUR PRODUCTION ET LEUR UTILISATION

(30) Priorität: 20.04.1998 DE 19817459
(43) Veröffentlichungstag der Anmeldung: 07.02.2001
(73) Patentinhaber: Abbott GmbH & Co. KG, 65205 Wiesbaden (DE)
(72) Erfinder: LUBISCH, Wilfried, D-69115 Heidelberg (DE); MÖLLER, Achim, D-67269 Grünstadt (DE); TREIBER, Hans-Jörg, D-68782 Brühl (DE); KNOPP, Monika, D-67071 Ludwigshafen (DE)
(74) Vertreter: Grünecker, Kinkeldey, Stockmair & Schwanhäusser Anwaltssozietät
(86) Internationale Anmeldenummer: PCT/EP1999/002611
(87) Internationale Veröffentlichungsnummer: WO 1999/054304

(56) Entgegenhaltungen:
- EP-A- 0 520 336
- WO-A-97/21690
- WO-A-98/41092
- WO-A-98/41506
- DE-A- 19 642 591

## Beschreibung

Die vorliegende Erfindung betrifft neuartige Amide, die Inhibitoren von Enzymen, insbesondere Cystein-Proteasen, wie Calpain (= Calcium dependant cysteine proteases) und dessen Isoenzyme und Cathepsine, zum Beispiel B und L, darstellen.

Calpaine stellen intracelluläre, proteolytische Enzyme aus der Gruppe der sogenannten Cystein-Proteasen dar und werden in vielen Zellen gefunden. Calpaine werden durch erhöhte Kalziumkonzentration aktiviert, wobei man zwischen Calpain I oder µ-Calpain, das durch µ-molare Konzentrationen von Calzium-Ionen aktiviert wird, und Calpain II oder m-Calpain, das durch m-molare Konzentrationen von Kalzium-Ionen aktiviert wird, unterscheidet (P.Johnson, Int.J.Biochem. 1990, 22(8), 811-22). Heute werden noch weitere Calpain-Isoenzyme postuliert (K.Suzuki et al., Biol.Chem. Hoppe-Seyler, 1995, 376(9),523-9).

Man vermutet, daß Calpaine in verschiedenen physiologischen Prozessen eine wichtige Rolle spielen. Dazu gehören Spaltungen von regulatorischen Proteinen wie Protein-Kinase C, Cytoskelett-Proteine wie MAP 2 und Spektrin, Muskelproteine, Proteinabbau in rheumatoider Arthritis, Proteine bei der Aktivierung von Plättchen, Neuropeptid-Metabolismus, Proteine in der Mitose und weitere, die in. M.J.Barrett et al., Life Sci. 1991, 48, 1659-69 und K.K.Wang et al., Trends in Pharmacol.Sci., 1994, 15, 412-9 aufgeführt sind.

Bei verschiedenen pathophysiologischen Prozessen wurden erhöhte Calpain-Spiegel gemessen, zum Beispiel: Ischämien des Herzens (z.B. Herzinfarkt), der Niere oder des Zentralnervensystems (z.B. "Stroke"), Entzündungen, Muskeldystrophien, Katarakten der Augen, Verletzungen des Zentralnervensystems ( z.B.Trauma), Alzheimer Krankheit usw.(siehe K.K. Wang, oben). Man vermutet einen Zusammenhang dieser Krankheiten mit erhöhten und anhaltenden intrazellulären Kalziumspiegeln. Dadurch werden Kalzium-abhängige Prozesse überaktiviert und unterliegen nicht mehr der physioogischen Regelung. Dementsprechend kann eine Überaktivierung von Calpainen auch pathophysiologische Prozesse auslösen.

Daher wurde postuliert, daß Inhibitoren der Calpain-Enzyme für die Behandlung dieser Krankheiten nützlich sein können. Verschiedene Untersuchungen bestätigen dies. So haben Seung-Chyul Hong et al., Stroke 1994, 25(3), 663-9 und R.T.Bartus et al., Neurological Res. 1995, 17, 249-58 eine neuroprotektive Wirkung von Calpain-Inhibitoren in akuten neurodegenerativen Störungen oder Ischämien, wie sie nach Hirnschlag auftreten, gezeigt. Ebenso nach experimentellen Gehirntraumata verbesserten Calpain-Inhibitoren die Erholung der auftretenden Gedächtnisleistungsdefizite und neuromotrischen Störungen (K.E.Saatman et al. Proc.Natl.Acad.Sci. USA, 1996, 93,3428-3433). C.L.Edelstein et al., Proc.Natl.Acad.Sci. USA, 1995, 92, 7662-6, fand eine protektive Wirkung von Calpain-Inhibitoren auf durch Hypoxie geschädigten Nieren. Yoshida, Ken Ischi et al., Jap.Circ.J. 1995, 59(1), 40-8, konnten günstige Effekte von Calpain-Inhibitoren nach cardialen Schädigungen aufzeigen, die durch Ischämie oder Reperfusion erzeugt wurden. Da Calpain-Inhibitoren die Freisetzung von dem β-AP4-Protein hemmen, wurde eine potentielle Anwendung als Therapeutikum der Alzheimer Krankheit vorgeschlagen (J.Higaki et al., Neuron, 1995, 14, 651-59). Die Freisetzung von Interleukin-1α wird ebenfalls durch Calpain-Inhibitoren gehemmt (N.Watanabe et al., Cytokine 1994, 6(6), 597-601). Weiterhin wurde gefunden, daß Calpain-Inhibitoren cytotoxische Effekte an Tumorzellen zeigen ( E.Shiba et al. 20th Meeting Int.Ass.Breast Cancer Res., Sendai Jp, 1994, 25.-28.Sept., Int.J.Oncol. 5 (Suppl.), 1994, 381).

Weitere mögliche Anwendungen von Calpain-Inhibitoren sind in K.K.Wang, Trends in Pharmacol.Sci., 1994, 15, 412-8, aufgeführt.

Calpain-Inhibitoren sind in der Literatur bereits beschrieben worden. Überwiegend sind dies jedoch entweder irreversible oder peptidische Inhibitoren. Irreversible Inhibitoren sind in der Regel alkylierende Substanzen und haben den Nachteil, daß sie im Organismus unselektiv reagieren oder instabil sind. So zeigen diese Inhibitoren oft unerwünschte Nebeneffekte, wie Toxizität, und sind danach in der Anwendung eingeschränkt oder nicht brauchbar. Zu den irreversiblen Inhibitoren kann man zum Beispiel die Epoxide E 64 (E.B.McGowan et al., Biochem.Biophys.Res.Commun. 1989, 158, 432-5), α-Halogenketone ( H.Angliker et al., J.Med.Chem. 1992, 35, 216-20) oder Disulfide (R.Matsueda et al., Chem.Lett. 1990, 191-194) zählen.

Viele bekannte reversible Inhibitoren von Cystein-Proteasen wie Calpain stellen peptidische Aldehyde dar, insbesondere dipeptidische und tripepidische Aldehyde wie zum Beispiel Z-Val-Phe-H (MDL 28170) (S.Mehdi, Tends in Biol.Sci. 1991, 16, 150-3). Unter physiologischen Bedingungen haben peptidische Aldehyde den Nachteil, daß sie auf Grund der großen Reaktivität häufig instabil sind, schnell metabolisiert werden können und zu unspezifischen Reaktionen neigen, die die Ursache von toxischen Effekten sein können ( J.A.Fehrentz und B.Castrc. Synthesis 1983, 676-78.

In JP 08183771 (CA 1996, 605307) und in EP 520336 sind Aldehyde, die sich von 4-Piperidinoylamide und 1-Carbonyl-piperidino-4-yl-amide ableiten, als Calpain-Inhibitoren beschrieben worden. In WO 97/21690 sind Aldehyde, die von N-Sulfonyl-prolinamide abgeleitet sind, hergestellt worden. In WO 96/06211 ist ein Aldehyd-Derivat analog zur allgemeinen Struktur I beschrieben, wobei allerdings Y ein Xanthin-Derivat darstellt, das jedoch keine weiteren Reste wie R¹-X trägt. Jedoch sind die hier beanspruchten Aldehyde, die sich von heteroaromatisch substituierten Amiden der allgemeinen Struktur I ableiten bisher noch nie beschrieben worden.

Peptidische Keton-Derivate sind ebenfalls Inhibitoren von Cystein-Proteasen, insbesondere Calpaine. So sind zum Beispiel bei Serin-Proteasen Keton-Derivate als Inhibitoren bekannt, wobei die Keto-Gruppe von einer elektronenziehenden Gruppe wie CF₃ aktiviert wird. Bei Cystein-Proteasen sind Derivate mit durch CF₃ oder ähnlichen Gruppen aktivierte Ketone wenig oder nicht wirksam (M.R.Angelastro et al., J.Med.Chem. 1990, 33, 11-13). Überraschenderweise konnten bei Calpain bisher nur Keton-Derivate, bei denen einerseits α-ständige Abgangsgruppen eine irreversible Hemmung verursachen und andererseits ein Carbonsäure-Derivat die Keto-Gruppe aktiviert, als wirksame Inhibitoren gefunden werden (siehe M.R.Angelastro et al., siehe oben; WO 92/11850; WO 92,12140; WO 94/00095 und WO 95/00535). Jedoch sind von diesen Ketoamiden und Ketoestern bisher nur peptidische Derivate als wirksam beschrieben worden (Zhaozhao Li et al., J.Med.Chem. 1993, 36, 3472-80;. S.L.Harbenson et al., J.Med.Chem. 1994, 37, 2918-29 und siehe oben M.R.Angelastro et al.).

Ketobenzamide sind bereits in der Literatur bekannt. So wurde der Ketoester PhCO-Abu-COOCH₂CH₃ in WO 94/00095 und WO 92/11850 beschrieben. Das analoge Phenyl-Derivat Ph-CONH-CH(CH₂Ph)-CO-COCOOCH₃ wurde in M.R.Angelastro et al., J.Med.Chem. 1990,33, 11-13 als jedoch nur schwacher Calpain-Inhibitor gefunden. Dieses Derivat ist auch in J.P.Burkhardt, Tetrahedron Lett., 1988. 3433-36 beschrieben. Die Bedeutung der substituierten Benzamide bzw. heterocyclischen Amiden ist jedoch bisher nie untersucht worden.

WO 98/41506 beschreibt Chinolin- und Naphthalincarboxamide, die Calpaine inhibitieren und zur Behandlung von beispielsweise neurodegenerativen Krankheiten eingesetzt werden.

WO 98/41092 beschreibt Indolcarboxamidderivate, die ebenfalls als Inhibitoren für Calpaine verwendet werden.

In der vorliegenden Erfindung wurden substitutierte nicht-peptidische Aldehyde, Ketocarbonsäuren und Ketoamid-Derivate beschrieben. Diese Verbindungen sind neu und zeigen überraschenderweise die Möglichkeit auf, durch Einbau von rigiden strukturellen Fragmenten potente nicht-peptidische Inhibitoren von Cystein-Proteasen, wie z.B. Calpain, zu erhalten.

Gegenstand der vorliegenden Erfindung sind heterozyklisch substituierte Amide der allgemeinen Formel I 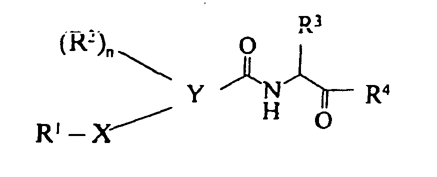 und ihre tautomeren Formen, möglichen enantiomeren und diastereomeren Formen, sowie mögliche physiologisch verträgliche Salze, worin die Variablen folgende Bedeutung haben:
- R¹: Phenyl, Naphthyl, Chinolyl, Pyridyl, Pyrimidyl, Pyrazyl, Pyridazyl, Imidazolyl, Thiazol, Chinazyl, Isochinolyl, Chinoxalyl, Thienyl, Benzothienyl, Benzofuranyl, Furanyl, und Indolyl bedeuten kann, wobei die Ringe noch mit zu bis 3 Resten R⁵ substituiert sein können,
- R²: Chlor, Brom, Fluor, C₁ - C₆ - Alkyl, C₁ - C₆ - Alkenyl, C₁ - C₆ - Alkinyl, C₁ - C₆-Alkyl-Phenyl, C₁ - C₆-Alkenyl-Phenyl, C₁ - C₆-Alkinyl-Phenyl, Phenyl, NHCO-C₁ -C₄-Alkyl, NHSO₂-C₁-C₄-Alkyl, -NHCOPhenyl, -NHCO-Naphthyl, NO₂,-O-C₁-C₄-Alkyl und NH₂ bedeutet, wobei die aromatischen Ringe noch ein oder zwei Reste R⁵ tragen können, und zwei Reste R² können zusammen auch eine Kette -CH=CH-CH=CH- darstellen und somit einen anellierten Benzo-Ring bilden, der seinerseits mit einem R⁵ substituiert sein kann und
- R³: -C₁-C₆-Alkyl, verzweigt oder unverzweigt, und der noch einen S-CH₃-Rest, Phenyl-, Cyclohexyl-, Cycloheptyl-, Cyclopentyl-, Indolyl-, Pyridyl- oder Naphthyl-Ring tragen kann, der seinerseits mit mit maximal zwei Resten R⁵ substituiert ist, wobei R⁵ Wasserstoff, C₁-C₄-Alkyl, verzweigt oder unverzweigt, -O-C₁-C₄-Alkyl, OH, Cl, F, Br, J, CF₃, NO₂ , NH₂ , CN , COOH , COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl, -(CH₂)ₙ-NR¹²R¹³ und -SO₂-Phenyl bedeutet,
- X: eine Bindung, -(CH₂)ₘ -, -(CH₂)ₘ -O-(CH₂)ₒ -, (CH₂)ₒ -S-(CH2)ₘ-, -(CH₂)ₒ -SO-(CH₂)ₘ -, -(CH₂)ₒ -SO₂-(CH₂)ₘ -, -CH=CH-, -C≡C-, -CO-CH=CH-, -(CH₂)ₒ-CO-(CH₂)ₘ-, -(CH₂)ₘ -NHCO-(CH₂)ₒ-, -(CH₂)ₘ - CONH-(CH₂)ₒ-, -(CH₂)ₘ -NFSO₂-(CH₂)ₒ-, -NH-CO-CH=CH-, -(CH₂)ₘ - SO₂NH-(CH₂)ₒ-, -CH=CH-CONH- und 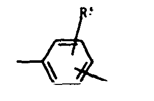 bedeutet,
und im Falle von CH=CH-Doppelbindungen sowohl die E als auch die Z-Form sein kann und
- R¹-X: zusammen auch 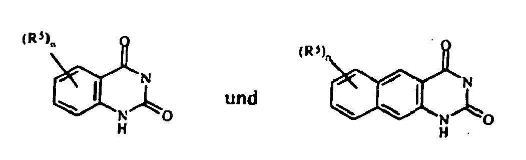 bedeuten und
- Y: Pyridin,
- R⁴: Wasserstoff, COOR⁶ und CO-Z bedeutet, worin Z NR⁷R⁸, und 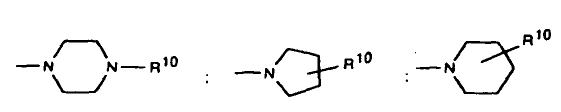 bedeutet,
- R⁶: Wasserstoff, C₁-C₆-Alkyl, geradlinig oder verzweigt, bedeutet und das mit einem Phenylring substituiert kann, der selbst noch mit einem oder zwei Resten R⁹ substituiert sein kann, und
- R⁷: Wasserstoff, C₁-C₆-Alkyl, verzweigt und unverzweigt, bedeutet, und
- R⁸: Wasserstoff, C₁-C₆-Alkyl, verzweigt oder unverzweigt, das noch mit einem Phenylring, der noch einen Rest R⁹ tragen kann, und mit 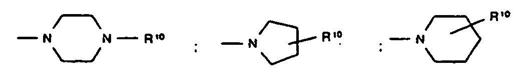 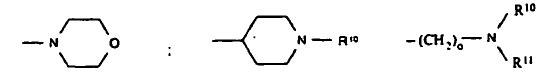 substituiert sein kann bedeutet, und
- R⁹: Wasserstoff, C₁-C₄-Alkyl, verzweigt oder unverzweigt, -O-C₁-C₄-Alkyl, OH Cl, F, Br, J, CF₃, NO₂ , NH₂ , CN , COOH , COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl und-SO₂-Phenyl bedeuten kann
- R¹⁰: Wasserstoff, C₁-C₆-Alkyl, geradlinig oder verzweigt, bedeutet und das mit einem Phenylring substituiert kann, der selbst noch mit einem oder zwei Resten R⁹ substituiert sein kann, und
- R¹¹: Wasserstoff, C₁-C₆-Alkyl, geradlinig oder verzweigt, bedeutet und das mit einem Phenylring substituiert kann, der selbst noch mit einem oder zwei Resten R⁹ substituiert sein kann, und
- n: eine Zahl 0, 1 oder 2 bedeutet, und
- m,o: unabhängig voneinander eine Zahl 0, 1, 2, 3 oder 4 bedeutet.

Die Verbindungen der Formel I können als Racemate, als enantiomerenreine Verbindungen oder als Diastereomere eingesetzt werden. Werden enantiomerereine Verbindungen gewünscht, kann man diese beispielweise dadurch erhalten, daß man mit einer geeigneten optisch aktiven Base oder Säure eine klassische Racematspaltung mit den Verbindungen der Formel I oder ihren Zwischenprodukten durchführt. Andereseits können die enantiomeren Verbindungen ebenfalls durch Einsatz von kommerziell erwerbbaren Verbindungen, zum Beispiel optisch aktive Aminosäuren wie Phenylalanin, Tryptophan und Tyrosin, hergestellt werden.

Gegenstand der Erfindung sind auch zu Verbindungen der Formel I mesomere oder tautomere Verbindungen, beispielweise solche, bei denen die Aldehyd- oder Ketogruppe der Formel I als Enol-Tautomeres vorliegt.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd.10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure, Äpfelsäure, Bernsteinsäure, Malonsäure, Schwefelsäure usw. bzw. Natriumhydroxid, Lithiumhydroxid,. Kaliumhydroxid, α,α,α-Tris(hydroxymethyl)methylamin, Triethylamin usw.

Die Herstellung der erfindungsgemäßen Amide I kann auf verschiedenen Wegen erfolgen, die im Syntheseschema skizziert wurde.

### Syntheseschema

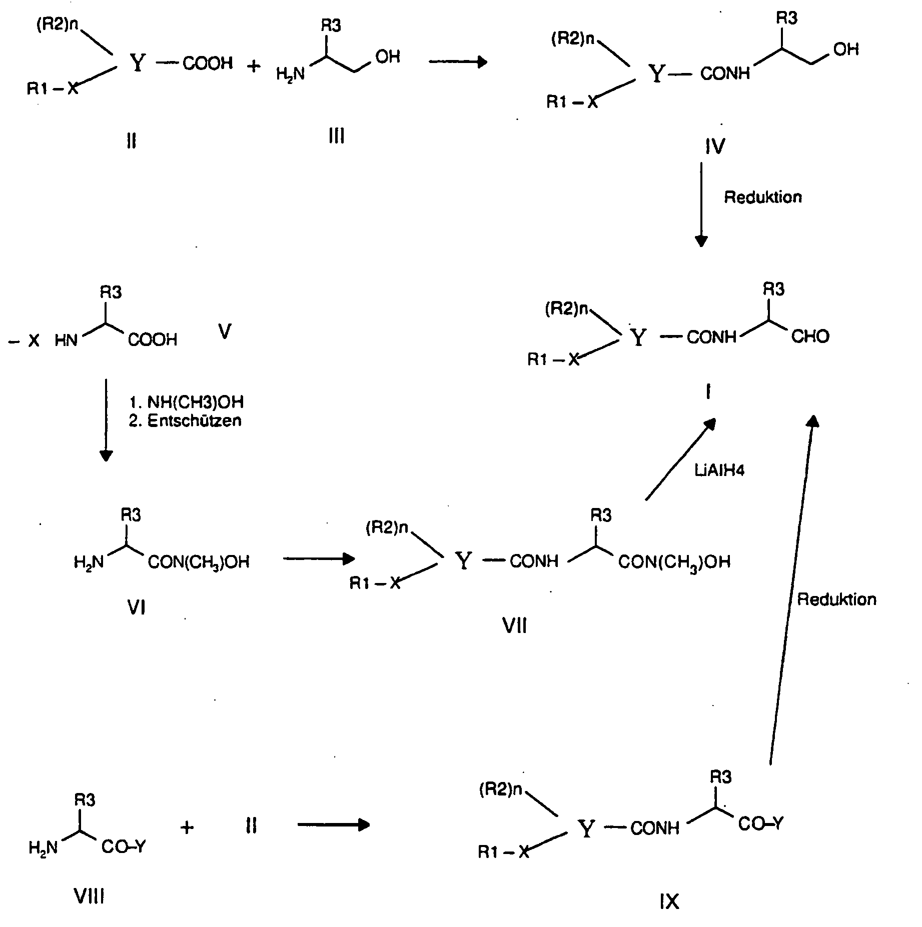

Heterocyclische Karbonsäuren II werden mit geeigneten Aminoalkoholen III zu den entsprechenden Amiden IV verknüpft. Dabei benutzt man übliche Peptid-Kupplungs-Methoden, die entweder im C.R.Larock, Comprehensive Organic Transformations, VCH Publisher, 1989, Seite 972f. oder im Houben-Weyl, Methoden der organischen Chemie, 4.Aufl., E5, Kap.V aufgeführt sind. Bevorzugt arbeitet man mit "aktivierten" Säurederivaten von II, wobei die Säuregruppe COOH in eine Gruppe COL überführt wird. L stellt eine Abgangsgruppe wie zum Beispiel C1, Imidazol und N-Hydroxybenzotriazol dar. Diese aktivierte Säure wird anschließend mit Aminen zu den Amiden IV umgesetzt. Die Reaktion erfolgt in wasserfreien, inerten Lösungsmitteln wie Methylenchlorid, Tetrahydrofuran und Dimethylformamid bei Temperaturen von -20 bis +25°C.

Diese Alkohol-Derivate IV können zu den erfindungsgemäßen Aldehyd-Derivaten I oxidiert werden. Dafür kann man verschiedene übliche Oxidationsreaktionen (siehe C.R.Larock, Comrenhensive Organic Transformations, VCH Publisher, 1989, Seite 604 f.) wie zum Beispiel Swern- und Swern-analoge Oxidationen ( T.T.Tidwell, Synthesis 1990, 857-70), Natriumhypochlorid/TEMPO ( S.L.Harbenson et al., siehe oben) oder Dess-Martin (J.Org.Chem. 1983, 48, 4155) benutzen. Bevorzugt arbeitet man hier in inerten aprotischen Lösungsmitteln wie Dimethylformamid, Tetrahydrofuran oder Methylenchorid mit Oxidationsmitteln wie DMSO/ py x SO₃ oder DMSO/ Oxalylchorid bei Temperaturen von -50 bis +25°C, je nach Methode (siehe obige Literatur).

Alternativ kann man die Karbonsäure II mit Aminohydroxamsäure-Derivate VI zu Benzamiden VII umsetzten. Dabei bedient man sich der gleichen Reaktionsfürung wie bei der Darstellung von IV. Die Hydroxam-Derivate VI sind aus den geschützten Aminosäuren V durch Umsatz mit einem Hydroxylamin erhältlich. Dabei benutzt man auch hier ein bereits beschriebenes Amidherstellungsverfahren. Die Abspaltung der Schutzgruppe X, zum Beispiel Boc, erfolgt in üblicher Weise, zum Beispiel mit Trifluoressigsäure. Die so erhaltenen Amid-hydroxamsäuren VII können durch Reduktion in die erfindungsgemäßen Aldehyde I umgewandelt werden. Dabei benutzt man zum Beispiel Lithiumaluminiumhydrid als Reduktionsmittel bei Temperaturen von -60 bis 0°C in inerten Lösungsmitteln wie Tetrahydrofuran oder Ether.

Analog zum letzten Verfahren kann man auch Karbonsäuren oder Säure-Derivate, wie Ester IX (Y = COOR', COSR') herstellen, die ebenfalls durch Reduktion in die erfindungsgemäßen Aldehyde I überführt werden können. Diese Verfahren sind in R.C.Larock, Comprehensive Organic Transformations, VCH Publisher, 1989, Seite 619-26 aufgelistet.

Die Herstellung der erfindungsgemäßen heterozyklisch substituierten Amide I, eine Ketoamid- oder Ketoester-gruppe tragen, kann auf verschiedenen Wegen erfolgen, die in den Syntheseschemata 2 und 3 skizziert wurden.

Gegebenenfalls werden die Karbonsäureester IIa mit Säuren oder Basen wie Lithiumhydroxid, Natriumhydroxid oder Kaliumhydroxid in wäßrigen Medium oder in Gemischen aus Wasser und organischen Lösungsmitteln wie Alkohole oder Tetrahydrofuran bei Raumtemperatur oder erhöhten Temperaturen, wie 25-100°C, in die Säuren II überführt.

Diese Säuren II werden mit einem α-Aminosäure-Derivat verknüpft, wobei man übliche Bedingungen benutzt, die zum Beispiel im Houben-Weyl, Methoden der organischen Chemie, 4.Aufl., E5, Kap. V, und C.R.Larock, Comprehensive Organic Transformations, VCH Publisher, 1989, Ch.9 aufgelistet sind.

Zum Beispiel werden die Carbonsäuren II in die "aktivierten" Säure-Derivate IIb =Y-COL überführt, wobei L eine Abgangsgruppe wie C1, Imidazol und N-Hydroxybenzotriazol darstellt und anschließend durch Zugabe von einem Aminosäure-Derivat H₂N-CH(R³)-COOR in das Derivat XI überführt. Diese Reaktion erfolgt in wasserfreien, inerten Lösungsmitteln wie Methylenchlorid, Tetrahydrofuran und Dimethylformamid bei Temperaturen von -20 bis +25°C.

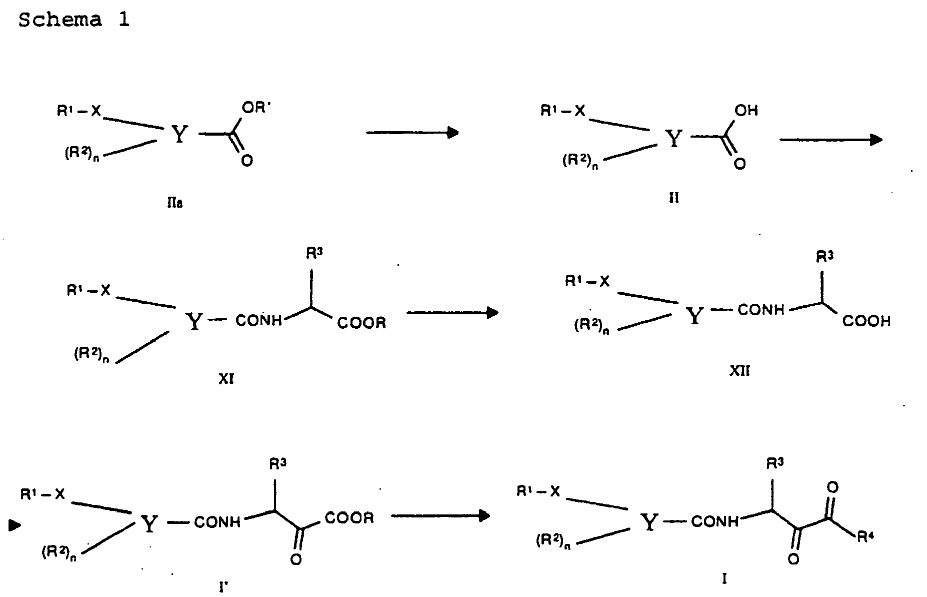

Die Derivate XI, die in der Regel Ester darstellen, werden analog der oben beschriebenen Hydrolyse in die Ketokarbonsäuren XII überführt. In einer Dakin-West analogen Reaktion werden die Ketoester I' hergestellt, wobei nach einer Methode von ZhaoZhao Li et al.. J.Med.Chem., 1993, 36, 3472-80 gearbeitet wird. Dabei werden Karbonsäuren wie XII bei erhöhter Temperatur (50-100°C) in Lösungsmitteln, wie zum Beispiel Tetrahydrofuran, mit Oxalsäuremonoesterchlorid umgesetzt und anschließend die so erhaltenen Produkte mit Basen wie Natriumethanolat in Ethanol bei Temperaturen von 25-80°C zu den erfindungsgemäßen Ketoestern I' umgesetzt. Die Ketoester I' können, wie oben beschrieben, zum Beispiel zu erfindungsgemäßen Ketocarbonsäuren hydrolysiert werden.

Die Umsetzung zu Ketobenzamiden I' erfolgt ebenfalls analog der Methode von ZhaoZhao Li et al.(s.oben). Die Ketogruppe in I' wird durch Zugabe von 1,2-Ethandithiol unter Lewissäure-Katalyse, wie zum Beispiel Bortrifluoridetherat, in inerten Lösungsmitteln, wie Methylenchlorid, bei Raumtemperatur geschützt, wobei ein Dithian anfällt. Diese Derivate werden mit Aminen R³-H in polaren Lösungsmitteln, wie Alkohole, bei Temperaturen von 0-80°C umgesetzt, wobei die Ketoamide I (R⁴ =Z oder NR⁷R⁸) anfallen.

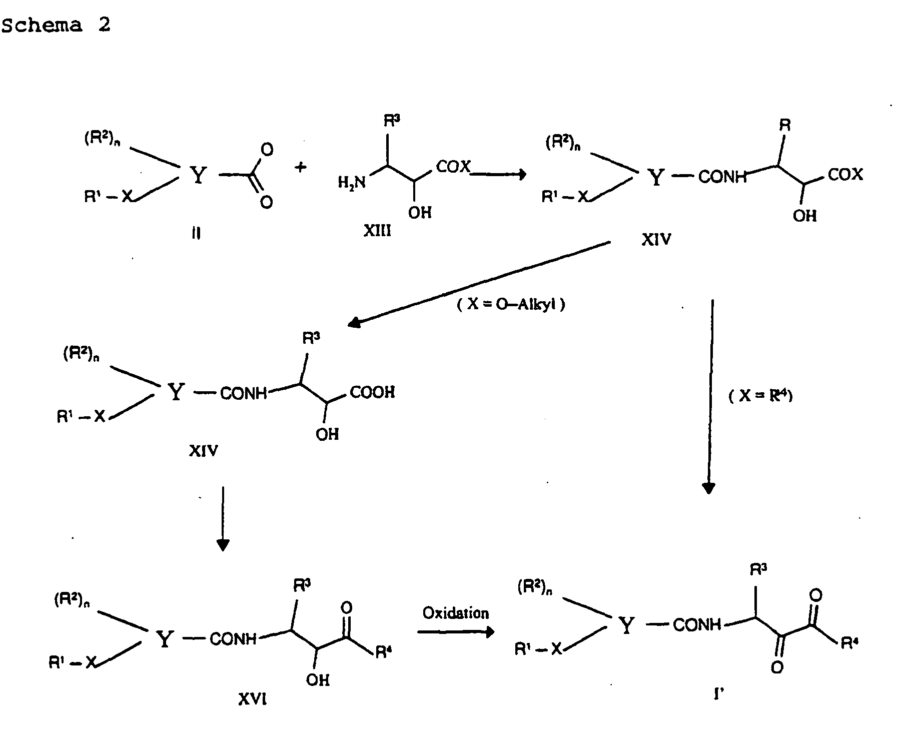

Eine alternative Methode ist im Schema 2 dargestellt. Die Ketokarbonsäuren II werden mit Aminohydroxykarbonsäure-Derivaten XIII ( Herstellung von XIII siehe s.L.Harbenson et al., J.Med.Chem. 1994, 37,2918-29 oder J.P. Burkhardt et al. Tetrahedron Lett. 1988, 29, 3433-3436) unter üblichen Peptid-Kupplungs-Methoden (siehe oben, Houben-Weyl) umgesetzt, wobei Amide XIV anfallen. Diese Alkohol-Derivate XIV können zu den erfindungsgemäßen Ketokarbonsäure-Derivaten I oxidiert werden. Dafür kann man verschiedene übliche Oxidationsreaktionen (siehe C.R.Larock, Comprehensive Organic Transformations, VCH Publisher, 1989, Seite 604 f.), wie zum Beispiel Swern- und Swern-analoge Oxidationen, bevorzugt Dimethylsulfoxid/ Pyridin-Schwefeltrioxid-Komplex in Lösungsmitteln wie Methylenchorid oder Tetrahydrofuran, gegebenenfalls unter Zusatz von Dimethylsulfoxid, bei Raumtemperatur oder Temperaturen von -50 bis 25°C, ( T.T.Tidwell, Synthesis 1990, 857-70) oder Natriumhypochlorid/TEMPO ( S.L.Harbenson et al., siehe oben), benutzen.

Wenn XIV α-Hydroxyester darstellen (X= O-Alkyl), können diese zu Karbonsäuren XV hydrolysiert werden, wobei analog zu den obigen Methoden gearbeitet wird, bevorzugt aber mit Lithiumhydroxid in Wasser/Tetrahydrofuran-Gemischen bei Raumtemperatur. Die Herstellung von anderen Estern oder Amiden XVI erfolgt durch Umsetzung mit Alkoholen oder Aminen unter bereits beschriebenen Kupplungsbedingungen. Das Alkohol-Derivat XVI kann erneut zu erfindungsgemäßen Ketokarbonsäure-Derivaten I oxidiert werden.

Die Herstellung der Karbonsäureester II sind teilweise bereits beschrieben worden oder erfolgen entsprechend üblichen chemischen Methoden.

Verbindungen, bei denen X eine Bindung darstellt, werden durch übliche aromatische Kupplung, zum Beispiel die Suzuki-Kupplung mit Borsäure-Derivaten und Halogenide unter Palladiumkatalyse oder Kupferkatalytische Kupplung von aromatischen Halogeniden, hergestellt. Die Alkyl-überbrückten Reste (X= -(CH₂)ₘ-) können durch Reduktion der analogen Ketone oder durch Alkylierung der Organolithium , z.B. ortho-Phenyloxazolidine, oder anderer Organometallverbindungen hergestellt werden (vgl. I.M.Dordor, et al., J.Chem.Soc. Perkin Trans. I, 1984, 1247-52).

Ether-überbrückte Derivate werden durch Alkylierung der entsprechenden Alkohole oder Phenole mit Halogeniden hergestellt.

Die Sulfoxide und Sulfone sind durch Oxidation der entsprechenden Thioether zugänglich.

Alken- und Alkin- überbrückte Verbindungen werden zum Beispiel durch Heck-Reaktion aus aromatischen Halogeniden und entsprechenden Alkenen und Alkinen hergestellt (vgl. I.Sakamoto et al., Chem.Pharm.Bull., 1986, 34, 2754-59).

Die Chalkone entstehen durch Kondensation aus Acetophenonen mit Aldehyden und können gegebenenfalls durch Hydrierung in die analogen Alkyl-Derivate überführt werden.

Amide und Sulfonamide werden analog den oben beschriebenen Methoden aus den Aminen und Säure-Derivaten hergestellt.

Die in der vorliegenden Erfindung enthaltenen heterozyklisch substituierte Amide I stellen Inhibitoren von Cystein-Proteasen dar, insbesondere Cystein-Proteasen wie die Calpaine I und II und Cathepsine B bzw. L.

Die inhibitorische Wirkung der heterozyklisch substituierte Amide I wurde mit in der Literatur üblichen Enzymtests ermittelt, wobei als Wirkmaßstab eine Konzentration des Inhibitors ermittelt wurde, bei der 50% der Enzymaktivität gehemmt wird ( = IC₅₀). Die Amide I wurden in dieser Weise auf Hemmwirkung von Calpain I, Calpain II und Cathepsin B gemessen.

### Cathepsin B-Test

Die Cathepsin B-Hemmung wurde analog einer Methode von S.Hasnain et al., J.Biol.Chem. 1993, 268, 235-40 bestimmt.

Zu 88µL Cathepsin B ( Cathepsin B aus menschlicher Leber (Calbiochem), verdünnt auf 5 Units in 500µM Puffer) werden 2µL einer Inhibitor-Lösung, hergestellt aus Inhibitor und DMSO (Endkonzentrationen: 100µM bis 0,01µM). Dieser Ansatz wird für 60 Minuten bei Raumtemperatur (25°C) vorinkubiert und anschließend die Reaktion durch Zugabe von 10µL 10mM Z-Arg-Arg-pNA (in Puffer mit 10% DMSO) gestartet. Die Reaktion wird 30 Minuten bei 405nM im Mikrotiterplattenreader verfolgt. Aus den maximalen Steigungen werden anschließend die IC₅₀'s bestimmt.

### Calpain I und II Test

Die Testung der inhibitorischen Eigenschaften von Calpain-Inhibitoren erfolgt in Puffer mit 50 mM Tris-HCl, pH 7,5 ; 0,1 M NaCl; 1 mM Dithiotreithol; 0,11 mM Ca Cl₂, wobei das fluorogene Calpainsubstrats Suc-Leu-Tyr-AMC ( 25 mM gelöst in DMSO, Bachem/Schweiz) verwendet wird. Humanes µ-Calpain wird aus Erythrozyten isoliert und nach mehren chromatographischen Schritten (DEAE-Sepharose, Phenyl-Sepharose, Superdex 200 und Blue-Sepharose) erhält man Enzym mit einer Reinheit >95%, beurteilt nach SDS-PAGE, Western Blot Analyse und N-terminaler Sequenzierung. Die Fluoreszenz des Spaltproduktes 7-Amino-4-methylcoumarin (AMC) wird in einem Spex-Fluorolog Fluorimeter bei λex = 380 nm und λem = 460 nm verfolgt. In einem Meßbereich von 60 min. ist die Spaltung des Substrats linear und die autokatalytische Aktivität von Calpain gering, wenn die Versuche bei Temperaturen von 12° C durchgeführt werden. Die Inhibitoren und das Calpainsubstrat werden in den Versuchsansatz als DMSO-Lösungen gegeben,, wobei DMSO in der Endkonzentration 2% nicht überschreiten soll.

In einem Versuchsansatz werden 10 µl Substrat (250µM final) und anschließend 10 µl an µ-Calpain (2µg/ml final, d.h.18 nM) in eine 1 ml Küvette gegeben, die Puffer enthält. Die Calpain-vermittelte Spaltung des Substrats wird für 15 - 20 min. gemessen. Anscließend Zugabe von 10 µl Inhibitor (50 - 100 µM Lösung in DMSO) und Messung der Inhibition der Spaltung für weitere 40 min.

Kᵢ -Werte werden nach der klassischen Gleichung für reversible Hemmung bestimmt:
Ki = I / (v0/vi) - 1 ; wobei I= Inhibitorkonzentration, v0 = Anfangsgeschwindigkeit vor Zugabe des Inhibitors; vi = Reaktionsgeschwindigkeit im Gleichgewicht.

Die Geschwindigkeit wird errechnet aus v = Freisetzung AMC/ Zeit d.h. Höhe /Zeit.

Calpain ist eine intrazelluläre Cysteinprotease. Calpain-Inhibitoren müssen die Zellmembran passieren, um den Abbau von intrazellulären Pproteinen durch Calpain zu verhindern. Einige bekannte Calpain-Inhibitoren, wie zum Beispiel E 64 und Leupeptin, überwinden die Zellmembranen nur schlecht und zeigen dementsprechend, obwohl sie gute Calpain-Inhibitoren darstellen, nur schlechte Wirkung an Zellen. Ziel ist es, Verbindungen mit besser Membrangängigkeit zu finden. Als Nachweis der Membrangängigkeit von Calpain-Inhibitoren benutzen wir humane Plättchen.

### Calpain-vermittelter Abbau der Tyrosinkinase pp60src in Plättchen

Nach der Aktivierung von Plättchen wird die Tyrosinkinase pp60src durch Calpain gespalten. Dies wurde von Oda et al. in J. Biol. Chem., 1993, Vol 268, 12603-12608 eingehend untersucht. Hierbei wurde gezeigt, daß die Spaltung von pp60src durch Calpeptin, einen Inhibitor für Calpain, verhindert werden kann. In Anlehnung an diese Publikation wurde die zellulare Effektivität unserer Substanzen getestet. Frisches humanes, mit Zitrat versetztes Blut wurde 15 min. bei 200g zentrifugiert. Das Plättchen-reiche Plasma wurde gepoolt und mit Plättchenpuffer 1:1 verdünnt ( Plättchenpuffer: 68 mM NaCl, 2,7 mM KCl, 0,5 mM MgCl₂ x 6 H₂O, 0,24 mM NaH₂PO₄ x H₂O, 12 mM NaHCO₃, 5,6 mM Glukose, 1 mM EDTA, pH 7,4). Nach einem Zentrifugations-und Waschschritt mit Plättchenpuffer wurden die Plättchen auf 10⁷Zellen/ml eingestellt. Die Isolierung der humanen Plättchen erfolgte bei RT.

Im Testansatz wurden isolierte Plättchen (2 x 10⁶ ) mit unterschiedlichen Konzentrationen an Inhibitoren (gelöst in DMSO) für 5 min. bei 37°C vorinkubiert. Anschließend erfolgte die Aktivierung der Plättchen mit 1µM Ionophor A23187 und 5 mM CaCl₂. Nach 5 min. Inkubation wurden die Plättchen kurz bei 13000 rpm zentrifugiert und das Pellet in SDS-Probenpuffer aufgenommen (SDS-Probenpuffer: 20 mM Tris-HCl, 5 mM EDTA, 5 mM EGTA, 1 mM DTT, 0,5 mM PMSF, 5 µg/ml Leupeptin, 10 µg/ml Pepstatin, 10% Glycerin und 1% SDS). Die Proteine wurden in einem 12%igen Gel aufgetrennt und pp60src und dessen 52-kDa und 47-kDa Spaltprodukte durch Western-Blotting identifiziert. Der verwendete polyklonale Kaninchen-Antikörper Anti-Cys-src (pp60^{c-src} ) wurde von der Firma Biomol Feinchemikalien (Hamburg) erworben. Dieser primäre Antikörper wurde mit einem HRP-gekoppelten zweiten Antikörper aus der Ziege (Boehringer Mannheim, FRG) nachgewiesen. Die Durchführung des Western-Blotting erfolgte nach bekannten Methoden.

Die Quantifizierung der Spaltung von pp60src erfolgte densitometrisch, wobei als Kontrollen nicht-aktivierte (Kontrolle 1: keine Spaltung) und mit Ionophor- und Kalzium-behandelte Plättchen (Kontrolle 2: entspricht 100% Spaltung) verwendet wurden. Der ED₅₀ -Wert entspricht der Konzentration an Inhibitor bei der die Intensität der Farbreaktion um 50% reduziert wird.

### Glutamat induzierter Zelltod an corticalen Neuronen

Der Test wurde, wie bei Choi D. W., Maulucci-Gedde M. A. and Kriegstein A. R., "Glutamate neurotoxicity in cortical cell culture". J. Neurosci. 1989, 7, 357-368, durchgeführt.

Aus 15 Tage alten Mäuseembryos wurden die Cortexhälften präpariert und die Einzelzellen enzymatisch (Trypsin) gewonnen. Diese Zellen (Glia und corticale Neuronen) werden in 24 Well-Platten ausgesät. Nach drei Tagen (Laminin beschichteten Platten) oder sieben Tagen (Ornithin beschichteten Platten) wird mit FDU (5-Fluor-2-Desoxyuridine) die Mitosebehandlung durchgeführt. 15 Tage nach der Zellpräparation wird durch Zugabe von Glutamat (15 Minuten) der Zelltod ausgelöst. Nach der Glutamatentfernung werden die Calpaininhibitoren zugegeben. 24 Stunden später wird durch die Bestimmung der Lactatdehydrogenase (LDH) im Zellkulturüberstand die Zellschädigung ermittelt.

Man postuliert, daß Calpain auch eine Rolle im apoptotischen Zelltod spielt ( M.K.T.Squier et al. J.Cell.Physiol. 1994, 159, 229-237; T.Patel et al. Faseb Journal 1996, 590, 587-597 ). Deshalb wurde in einem weiteren Modell in einer humanen Zellinie der Zelltod mit Kalzium in Gegenwart eines Kalziumionophors ausgelöst. Calpain-Inhibitoren müssen in die Zelle gelangen und dort Calpain hemmen, um den ausgelösten Zelltod zu verhindern.

### Kalzium-vermittelter Zelltod in NT2 Zellen

In der humanen Zellinie NT2 (Vorläufer-Zellen, Strategene GmbH) läßt sich durch Kalzium in Gegenwart des Ionophors A 23187 der Zelltod auslösen. 10⁵ Zellen/well wurden in Mikrotiterplatten 20 Stunden vor dem Versuch ausplattiert. Nach diesem Zeitraum wurden die Zellen mit verschiedenen Konzentrationen an Inhibitoren in Gegenwart von 2,5 µM Ionophor und 5 mM Kalzium inkubiert. Dem Reaktionsansatz wurden nach 5 Stunden 0,05 ml XTT (Cell Proliferation Kit II, Boehringer Mannnheim ) hinzugegeben. Die optische Dichte wird ungefähr 17 Stunden später, entsprechend den Angaben des Herstellers, in dem Easy Reader EAR 400 der Firma SLT bestimmt. Die optische Dichte, bei der die Hälfte der Zellen abgestorben sind, errechnet sich aus den beiden Kontrollen mit Zellen ohne Inhibitoren, die in Abwesenheit und Gegenwart von Ionophor inkubiert wurden.

Bei einer Reihe von neurologischen Krankheiten oder:psychischen Störungen treten erhöhte Glutamat-Aktivitäten auf, die zu Zuständen von Übererregungen oder toxischen Effekten im zentralen Nervensystem (ZNS) führen. Glutamat vermittelt seine Effekte über verschiedene Rezeptoren. Zwei von diesen Rezeptoren:werden nach den spezifischen Agonisten NMDA-Rezeptor und AMPA-Rezeptor klassifiziert. Substanzen, die diese von Glutamat ausgelöstenen Effekte abschwächen, können somit zur Behandlung dieser Krankheiten eingesetzt werden, insbesondere zur therepeutischen Anwendung gegen neurodegenerativen Krankeiten wie Chorea Huntington und Parkinsonsche Krankheit, neurotoxischen Störungen nach Hypoxie, Anoxie, Ischämie und nach Lesionen, wie sie nach Schlaganfall und Trauma auftreten, oder auch als Antiepileptika (vgl. Arzneim.Forschung 1990, 40, 511-514; TIPS, 1990, 11, 334-338; Drugs of the Future 1989, 14, 1059-1071).

Schutz gegen zerebrale Übererregung durch exzitatorische Aminosäuren ( NMDA- bzw. AMPA-Antagonismus an der Maus)

Durch intrazerebrale Applikation von exzitatorischen Aminosäuren EAA ( Excitatory Amino Acids) wird eine so massive Übererregung induziert, daß diese in kurzer Zeit zu Krämpfen und zum Tod der Tiere(Maus) führt. Durch systemische, z.B. intraperitoneale, Gabe von zentral-wirksamen Wirkstoffen (EAA-Antagonisten) lassen sich diese Symptome hemmen. Da die excessive Aktivierung von EAA-Rezeptoren des Zentralnervensystems in der Pathogenese verschiedener neurologischer Erkrankungen eine bedeutende Rolle spielt, kann aus dem nachgewiesenen EAA-Antagonismus in vivo auf eine mögliche therapeutische Verwendbarkeit der Substanzen gegen derartige ZNS-Erkrankungen geschlossen werden. Als Maß für die Wirksamkeit der Substanzen wurde ein ED₅₀-Wert bestimmt, bei dem 50% der Tiere durch eine festgelegte Dosis von entweder NMDA oder AMPA durch die vorangegangene ip.-Gabe der Meßsubstanz syptomfrei werden.

Es wurde bereits gezeigt, daß auch Calpain-Inhibitoren in Zellkulturen protektive Wirkung gegen den durch EAA ausgelösten Zelltod zeigen (H.Cauer et al., Brain Research 1993, 607, 354-356; Yu Cheg and A.Y. Sun, Neurochem. Res. 1994, 19, 1557-1564). Die in dieser Anmeldung enthaltenen Calpain-Inhibitoren sind überrascehnderweise sogar gegen die durch EAA (z.B. NMDA oder AMPA) ausgelösten Krämpfe *in vivo* (Maus) wirksam und zeigen damit auf eine mögliche therapeutische Verwendung in den oben genannten ZNS-Erkrankungen an.

Die heterozyklisch substituierten Amide I stellen Inhibitoren von Cystein-Derivate wie Calpain I bzw. II und Cathepsin B bzw. L dar und können somit zur Bekämpfung von Krankheiten, die mit einer erhöhten Enzymaktivität der Calpain-Enzyme oder Cathepsin-Enzyme verbunden sind, dienen. Die vorliegenden Amide I können danach zur Behandlung von neurodegenerativen Krankheiten, die nach Ischämie, Trauma, Subarachnoidal-Blutungen und Stroke auftreten, und von neurodegenerativen Krankheiten wie multipler Infarkt-Dementia, Alzheimer Krankheit, Huntington Krankheit und von Epilepsien und weiterhin zur Behandlung von Schädigungen des Herzens nach cardialen Ischämien, Reperfusionsschädigungen nach Gefäßverschlüssen, Schädigungen der Nieren nach renalen Ischämien, Skelettmuskelschädigungen, Muskeldystrophien, Schädigungen, die durch Proliferation der glatten Muskelzellen entstehen, coronaren Vasospasmen, cerebralen Vasospasmen, Katarakten der Augen, Restenosis der Blutbahnen nach Angioplastie dienen. Zudem können die Amide I bei der Chemotherapie von Tumoren und deren Metastasierung nützlich sein und zur Behandlung von Krankheiten, bei denen ein erhöhter Interleukin-1-Spiegel auftritt, wie bei Entzündungen und rheumatischen Erkrankungen, dienen.

Die erfindungsgemäßen Arzneimittelzubereitungen enthalten neben den üblichen Arneimittelhilfstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wikstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verbreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiele

### Beispiel 1

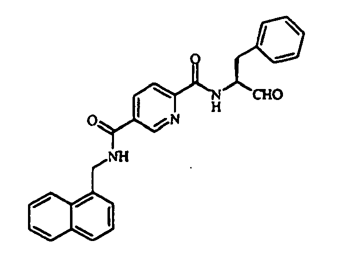

### (S)-4(N(1-Naphthylmethyl)carbamoyl)-N(3-phenyl-propan-1-al-2-yl)-pyridin-2-carbon-säureamid

### a) 4(N(1-Naphthylmethyl)carbamoyl)-pyridin-2-carbonsäureethylester

4.9g (25mMol) der 2-Ethoxycarbonylpyridin-3-carbonsäure ( N.Finch et al., J.Med.Chem. 1980, 23, 1405) wurden in 110ml Tetrahydrofuran/Dimethylformamid (10/1) gelöst und mit 4.5g (27.5mMol) 1,1'-Carbonyl-diimidazol versetzt. Nachdem man 30 min. bei Raumtemperatur gerührt hatte, fügte man noch 3.9g (25mMol) 1-Aminomethylnaphthalin zu und rührte weitere 72h bei Raumtemperatur. Anschließend wurde das Tetrahydrofuran im Vakuum entfernt und der Rückstand zwischen 200ml Essigester und 200ml wäßriger Natriumhydrogencarbonat-Lösung verteilt. Die organische Phase wurde noch mit Wasser gewaschen , getrocknet und im Vakuum eingeengt. Man erhielt 7.9g (95%) des Produktes.
1H-NMR:

### b) 4(N(1-Naphthylmethyl)carbamoyl)-pyridin-2-carbonsäure

6.9g (20mMol) des Zwischenproduktes 1a wurden in 100ml Ethanol gelöst und mit 3.3g (82mMol) Natriumhydroxid, gelöst in 50ml Wasser, versetzt. Alles wurde 16h bei Raumtemperatur gerührt. Anschließend wurde die Reaktionslösung mit 1M Salzsäure neutralisiert und das Ethanol im Vakuum entfernt. Der anfallende Niederschlag wurde abgesaugt und getrocknet. Man erhielt 5.6g (89%) des Produktes.

### c) (S)-4(N(1-Naphthylmethyl)carbamoyl)-N(3-phenyl-propan-1-ol-2-yl)-pyridin-2-carbon-säureamid

2.7g (9mMol) des Zwischenproduktes 1b und 1,4g (9mMol) (S)-Phenylalaninol wurden in 60ml Methylenchlorid gegeben und mit 2.3g( 22.5mMol) Triethylamin, 50ml Dimethyl-formamid und 0.4g (3mMol) 1-Hydroxybenzotriazol versetzt. Anschließend wurde bei 0°C 1.7g (9mMol) 1-Ethyl-3-(dimethylaminopropyl)carbodiimidhydrochlorid zugegeben und alles für 16h zuerst bei 0°C, dann bei Raumtemperatur gerührt. Der Reaktionsansatz wurde nacheinander mit 100ml 5%iger Zitronensäure und 100ml Natriumhydrogenkarbonat-Lösung gewaschen und, nach dem Trocknen, im Vakuum eingeengt. Man erhielt 2.4g (62%) des Produktes.

### d) (S)-4(N(1-Naphthylmethyl)carbamoyl)-N(3-phenyl-propan-1-al-2-yl)-pyridin-2-carbon-säureamid

1.9g (4.4mMol) der Zwischenverbindung 1c wurden in 50ml trockenem Dimethylsulfoxid gelöst und mit einer Lösung aus 1.8g (17,4mMol) Triethylamin und 2.8g (17.4mMol) Pyridin-Schwefeltrioxid-Komplex in 50ml trockenem Dimethylsulfoxid versetzt. Alles wurde für 16h bei Raumtemperatur gerührt. Anschließend wurde der Reaktiosansatz auf Wasser gegeben und der Niederschlag abgesaugt. Man erhielt 1.5g (80%) des Produktes.
1H-NMR (D₆-DMSO): δ = 3.1 (1H), 3.5(1H), 4.7(1H), 5.1(1H), 7.1-7.3(6H), 7.4-7.7(5H), 7.9(1H), 7.95(1H), 8.15(1H), 8.2(1H), 8.4(1H), 9.1(1H), 9.2(1H), 9.4(1H) und 9.8(1H)ppm.

### Beispiel 2

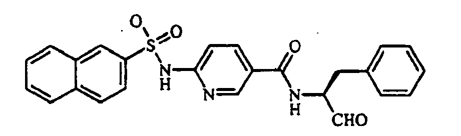

### (S)-2(2-Naphthalinsulfonamido)-N(3-phenyl-propan-1-al-2-yl)-pyridin-3-carbonsäure-amid

### a) 2 (2-Naphthalinsulfonamido)-pyridin-3-carbonsäuremethylester

Zu 4.7g (25mMol) 6-Aminonicotinsäuremethylesterhydrochlorid in 100ml trockenem Pyridin wurden bei Raumtemperatur 5.9g (26mMol) Naphthalin-2-sulfonsäurechlorid portionsweise zugegeben. Danach wurde alles für 16h bei Raumtemperatur gerührt. Die Reaktionslösung wurde dann auf 500ml Wasser gegossen und der angefallene Niederschlag abgesaugt. Man erhielt 6.4g (75%) des Produktes.

### b) 2(2-Naphthalinsulfonamido)-pyridin-3-carbonsäure

6g (17mMol) der Zwischenverbindung 2a, gelöst in 100ml Methanol, wurden mit 4,2g (104mMol) Natriumhydroxid, gelöst in 100ml Wasser, bei Raumtemperatur für 16h gerührt. Anschließend wurde das organische Lösungsmittel im Vakuum entfernt und der anfallende wäßrige Lösung mit 1M Salzsäure neutralisiert. Der enstandene Niederschlag wurde abgesaugt. Man erhielt 5.1g (90%) des Produktes.

### c) (S)-2(2-Naphthalinsulfonamido)-N(3-phenyl-propan-1-ol-2-yl)-pyridin-3-carbonsäureamid

2.5g (7.5mMol) der Zwischenverbindung 2b wurde analog der Vorschrift 1c mit (S)-Phenyl-alaninol umgesetzt. Man erhielt 0.7g (21%) des Produktes.

### d) (S)-2(2-Naphthalinsulfonamido)-N(3-phenyl-propan-1-al-2-yl)-pyridin-3-carbonsäureamid

0.5g (1.2mMol) der Zwischenverbindung 2c wurden analog der Vorschrift 1d oxidiert, wobei 0.4g (78%) des Produktes anfielen. 1H-NMR (D₆-DMSO): δ = 2.8(1H), 3.3(1H), 4.5(1H), 7.0-7.4(5H), 7.7(2H), 7.9(1H), 8.1(3H), 8.25(1H), 8.5(1H), 8.7(1H), 8.9(1H), 9.6(1H) und ca. 12.5(breit,1H) ppm.

### Beispiel 3

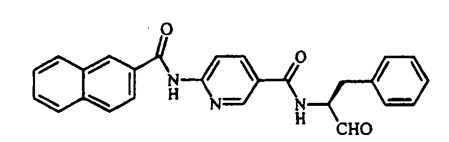

### (S)-2(2-Naphthalinamido)-N(3-phenyl-propan-1-al-2-yl)-pyridin-5-carbonsäureamid

### a) 6-Aminonicotinsäurehydrochlorid

20g (0.145Mol) 6-Aminonicotinsäure wurden in einem Gemisch aus 200ml Methanol 250ml 2.5M Salzsäure für etwa 5h unter Rückfluß gekocht. Danach wurde alles im Vakuum einggeengt und man erhielt 26.6g (97%) des Produktes.

### b) 6(2-Naphthalinamido)-nicotinsäuremethylester

4.7g (25mMol) der Zwischenverbindung 3a wurden in 100ml Pyridin gelöst und bei Raumtemperatur portionsweise mit 5g (25mol) 2-Naphthoylchlorid versetzt. Alles wurde für 16h bei Raumtemperatur gerührt. Danach wurde das Reaktionsgemisch auf Wasser gegossen und der anfallende Niederschlag abgesaugt. Man erhielt 5.4g (70%) des Produktes.

### c) 6(2-Naphthalinamido)-nicotinsäure

4.7g (15mMol) der Zwischenverbindung 3b wurden in 75ml Ethanol gelöst und mit 2.5g Natriumhydroxid, gelöst in 50ml Wasser, versetzt. Alles wurde für 16h bei Raumtemperatur gerührt. Anschließend wurde das Ethanol im Vakuum entfernt und der wäßrige Rückstand mit 1M Salzsäure neutralisiert. Der anfallende Niederschlag wurde abgesaugt. Man erhielt 3.1g (69%) des Produktes.

### d) (S)-2(2-Naphthalinamido)-N(3-phenyl-propan-1-ol-2-yl)-pyridin-5-carbonsäure-amid

2.7g (9.2mMol) der Zwischenverbindung 3c wurden analog der Vorschrift 1c mit (S)-Phenyl-alaninol umgesetzt. Man erhielt 2.1g (54%) des Produktes.

### e) (S)-2(2-Naphthalinamido)-N(3-phenyl-propan-1-al-2-yl)-pyridin-5-carbonsäureamid

1.7g (4mMol) der Zwischenverbindung 3d wurden analog der Vorschrift 1d oxidiert. Man erhielt 1.3g (79%) des Produktes. MS : m/e = 423 (M⁺).

### Beispiel 4

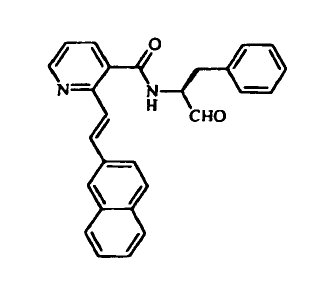

### (S)-2(2-Naphthyl)ethen-1-yl-N(3-phenyl-propan-1-al-2-yl)-pyridin-3-carbonsäureamid

### a) 2(2-Naphthyl-ethen-1-yl)-pyridin-3-carbonsäureethylesterhydrochlorid

10g (43.5mMol) 2-Brompyridine-3-carbonsäureethylester, 8.7g(56.5mMol) 2-Vinylnapththalin, 15ml (0.11Mol) Triethylamin, 0.36g Palladium-II-acetat und 0.96g Tri-o-toluidinphosphin wurden in 150ml Dimethylformamid gelöst. Man gab anschließend noch 1ml Wasser hinzu und kochte alles für 3h unter Rückfluß. Danach wurde alles mit Ether extrahiert. Die organische Phase wurde noch mit Wasser gewaschen, getrocknet und im Vakuum eingeengt. Der Rückstand wurde in Aceton gelöst und mit Chlorwasserstoff, gelöst in Dioxan, versetzt. Durch Zugabe von Ether wurde anschließend das Produkt ausgefällt. Man erhielt 8.7g (67%) des Produktes.

### b) 2(2-Naphthyl-ethen-1-yl)-pyridin-3-carbonsäure

8.5g (28mMol) des Zwischenproduktes 13a wurden in 70ml Tetrahydrofuran gelöst und mit 140ml 2M Natronlauge versetzt. Alles wurde für 8h unter Rückfluß gekocht. Anschließend wurde der Ansatz auf Eiswasser gegossen und mit Essigsäure neutralisiert. Das langsam auskristallisierende Produkt wurde abgesaugt und getrocknet. Man erhielt 5.6g (73%) des Produktes.

### c) (S)-2(2-Naphthyl)ethen-1-yl-N(3-phenyl-propan-1-ol-2-yl)-pyridin-3-carbonsäureamid

2g (7.3mMol) des Zwischenproduktes 13b und 1.1g (7.3mMol) (2S),(3R,S)-3-Amino-2-hydroxy-3-phenyl-buttersäureamid-trifluoracetat wurden analog der Vorschrift 1c umgesetzt. Man erhielt 2.1g (71%) des Produktes.

### d) (S)-2(2-Naphthyl)ethen-1-yl-N(3-phenyl-propan-1-al-2-yl)-pyridin-3-carbonsäureamid

1.9g (4.7mMol) der Zwischenverbindung 13c wurden analog der Vorschrift 1d oxidiert. Man erhielt 0.56g (30%) des Produktes.
MS : m/e = 406 (M⁺).

### Beispiel 5

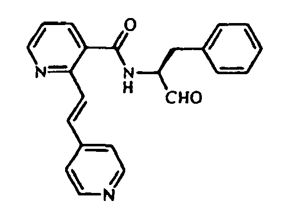

### (S)-N(3-Phenyl-propan-1-al-2-yl)-2(-4-pyridyl)-ethen-1-yl-pyridin-3-carbonsäureamid

### a) 2(4-Pyridine)-ethen-1-yl-pyridin-3-carbonsäureethylester

11.5g (49.9mMol) 2-Brompyridine-3-carbonsäureethylester und 6.8g(64.9mMol) 4-Vinylpyridin wurden analog der Vorschrift 5 13a umgesetzt. Man erhielt 7.0g (49%) des Produktes.

### b) 2(4-Pyridyl)-ethen-1-yl-pyridin-3-carbonsäure

7.0g (27.5mMol) des Zwischenproduktes 14a wurden in 50ml Tetrahydrofuran gelöst und mit 100ml 2M Natronlauge versetzt. Alles wurde für 2h unter Rückfluß gekocht. Anschließend wurde das organische Lösungsmittel im Vakuum entfernt und die anfallende wäßrige Phase mit Essigsäure angesäuert. Die wäßrige Phase wurde eingeengt und der Rückstand chromatographisch (Fließmittel: Essigester/Methanol/Essigsäure = 50/50/1)d gereinigt. Man erhielt 5.5g (89%) des Produktes.

### c) (S)-N(3-Phenyl-propan-1-ol-2-yl)-2(4-pyridyl)ethen-1-yl-pyridin-3-carbonsäureamid

1.5g (6.6mMol) des Zwischenproduktes 14b und 1.0g (6.6mMol) (S)-2-Amino-3-phenyl-propanol wurden analog der Vorschrift 1c umgesetzt. Man erhielt 1.7g (72%) des Produktes.

### d) (S)-N(3-Phenyl-propan-1-al-2-yl)-2(4-pyridyl)ethen-1-yl-pyridin-3-carbonsäureamid

1.5g (4.2mMol) der Zwischenverbindung 14c wurden analog der Vorschrift 1d oxidiert. Man erhielt 0.71g (48%) des Produktes.
MS : m/e = 357 (M⁺).

Folgende Beispiele wurden analog den obigen Beispielen und Vorschriften hergestellt:

### Beispiel 6

### (S)-N(3-Phenyl-propan-1-al-2-yl)-2(4-pyridyl)-chinolin-4-carbonsäureamid

¹H-NMR (d₆-DMSO):δ = 3.0(1H), 3.4(1H), 4.8(1H), 7.25(1H), 7.7(2H), 7.9(2H), 8.1(1H9, 8.25(1H), 8.7(1H), 9.0(1H), 9.5(1H9, und 9.8(1H),ppm.

### Beispiel 7

### (S)-N(3-Phenyl-propan-1-al-2-yl)-2-(2-pyridyl)-chinolin-4-carbonsäureamid

¹H-NMR(D₆-DMSO) :δ = 2.9(1H), 3.3(1H9, 4.8(1H),7.2-(.2(11H), 8.5(1H), 8.6(1H), 8.8(1H), 9.4(1H) und 9.0(1H)ppm.

### Beispiel 8

### N(1-Carbamoyl-1-oxo-3-phenyl-propan-2-yl)-2-(2-pyridyl)-chinolin-4-carbonsäureamid

MS: m/e=424 (M⁺).

### Beispiele 9

### N(1-Carbamoyl-1-oxo-3-phenyl-propan-2-yl)-2(E-2(4-pyridyl)-ethen-1-yl)-pyridin-3-carbonsäureamid

¹H-NMR(CF₃COOD):δ=3.1(1H), 3.7(1H), 6.1(1H), 7.1-7.6(5H), 8.0(1H), 8.1-8.5(4H9, 8.6(1H), 9.0(2H) und 9.1(1H)ppm.

### Beispiel 10

### N(1-Carbamoyl-1-oxo-3-phenyl-propan-2-yl)-2-(2-pyridyl)-chinolin-4-carbonsäureamid

MS: m/e=424(M⁺).

### Beispiel 11

### N(1-Carbamoyl-1-oxo-3-phenyl-propan-2-yl)-2-(1,2,3,4-tetrahydroisochinolin-2yl)-pyridin-3-carbonsäureamid

¹H-NMR(D₆-DMSO):δ=2.8(2H), 2.9(1H), 3.2(2H), 3.3(1H), 4.3(1H), 5.3(1H), 6.8(1H), 7.0-7.5(9H), 7.5(1H), 7.9-8.1(2H) und 9.0(1H)ppm.

### Beispiel 12

### N(1-Carboyl-1-oxo-3-phenyl-propan-2-yl)-2(6,7-dimethoxy-1,2,3,4-tetrahydroisochinolin-2yl)-pyridin-3-carbonsäureamid

¹H-NMR (D₆-DMSO):δ=2.7(2H), 2.8(1H), 3.2(2H), 3.4(1H), 3.7(6H), 4.2(1H), 5.3(1H9, 6.7((1H), 6.95(1H), 7.1-7.5(&H), 7.9(1H), 8.1(1H), 8.4(1H), 9.0(1H)ppm.

### Beispiel 13

### N(1-Carbamoyl-1-oxo-3-phenyl-propan-2-yl)-2-(3-phenylpyrrolidin-1-yl)-pyridin-3-carbonsäureamid

¹H-NMR(CF₃COOD):δ=2.0-2.7(2H), 2.95(1H), 3.3-4.0(6H), 5.9(1H), 6.9(1H), 7.0-7.5(10H) und 7.9(1H)ppm.

| | | | | | |
|---|---|---|---|---|---|
| 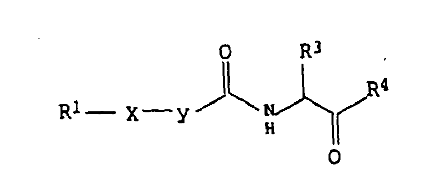 | | | | | |

| Beispiel | R¹ | X | Y | R³ | R⁴ |
|---|---|---|---|---|---|
| 15 | 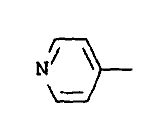 | 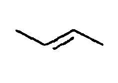 | 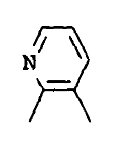 | CH₂Ph | CONH₂ |
| 16 | 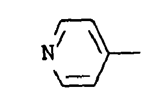 | 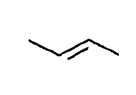 | 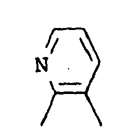 | CH₂Ph | 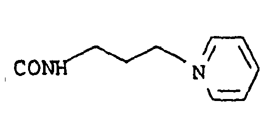 |
| 17 | 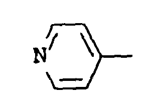 | 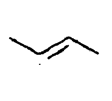 | 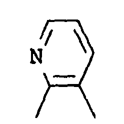 | CH₂Ph | 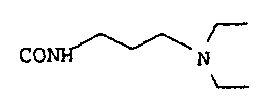 |
| 18 | 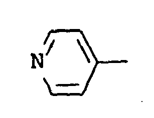 | 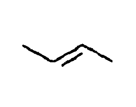 | 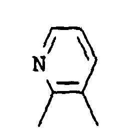 | CH₂Ph | 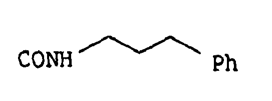 |
| 19 | 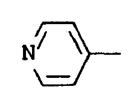 | 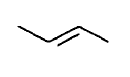 | 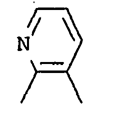 | (CH₂)₃-CH₃ | CONH₂ |
| 20 | 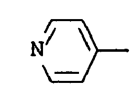 | 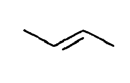 | 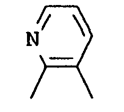 | (CH₂)₃-CH₃ | H |
| 21 | 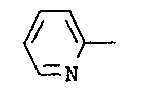 | 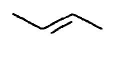 | 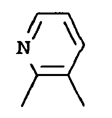 | CH₂Ph | H |
| 22 | 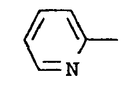 | 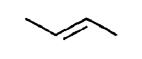 | 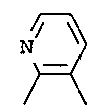 | CH₂Ph | CONH₂ |
| 23 | 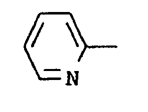 | 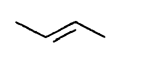 | 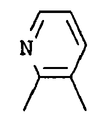 | (CH₂)₃-CH₃ | CONH₂ |
| 24 | 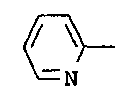 | 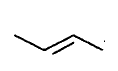 | 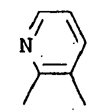 | (CH₂)₃-CH₃ | H |
| 25 | 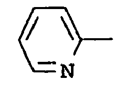 | | 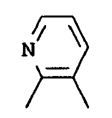 | CH₂Ph | H |
| 26 | 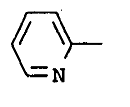 | | 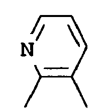 | CH₂Ph | CONH₂ |
| 27 | 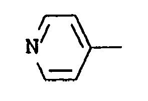 | | 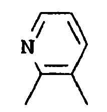 | CH₂Ph | CONH₂ |
| 28 | 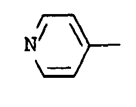 | | 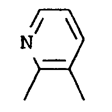 | CH₂Ph | H |
| 29 | 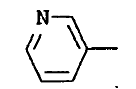 | | 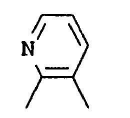 | CH₂Ph | H |
| 30 | 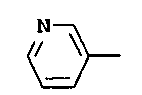 | | 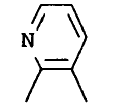 | CH₂Ph | CONH₂ |
| 31 | 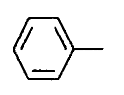 | 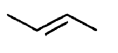 | 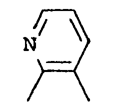 | CH₂Ph | H |
| 32 | 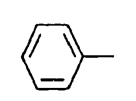 | 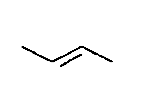 | 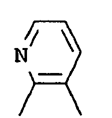 | CH₂Ph | CONH₂ |
| 33 | 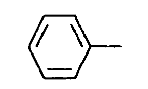 | 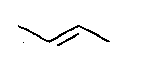 | 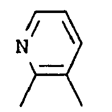 | (CH₂)₃CH₃ | H |
| 34 | 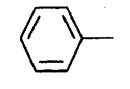 | 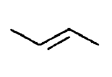 | 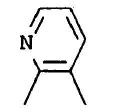 | (CH₂)₃CH₃ | CONH₂ |
| 35 | 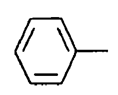 | 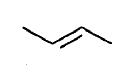 | 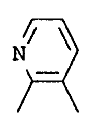 | CH₂Ph | 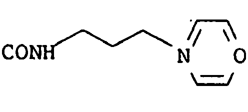 |
| 36 | 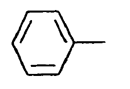 | 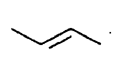 | 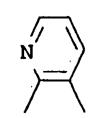 | | 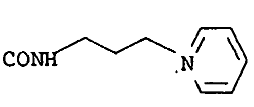 |
| 37 | 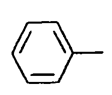 | 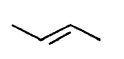 | 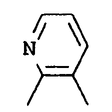 | CH₂Ph | |
| 38 | 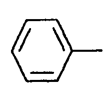 | 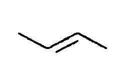 | 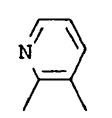 | CH₂Ph | 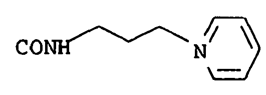 |
| 39 | 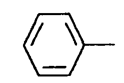 | 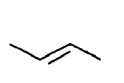 | 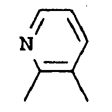 | CH₂Ph | 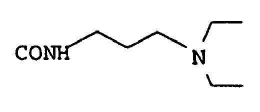 |
| 40 | 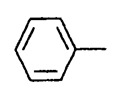 | 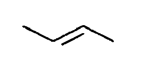 | 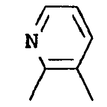 | CH₂Ph | 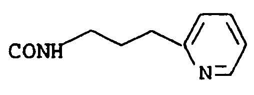 |
| 41 | 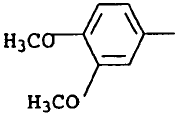 | 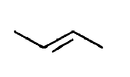 | 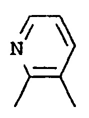 | CH₂Ph | H |
| 42 | 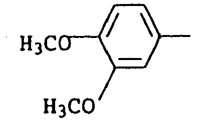 | 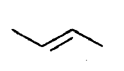 | 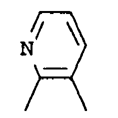 | CH₂Ph | CONH₂ |
| 43 | 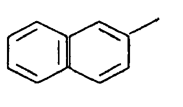 | -SO₂NH- | 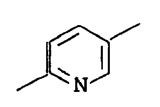 | CH₂Ph | H |
| 44 | 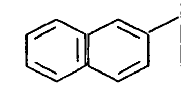 | -SO₂NH- | 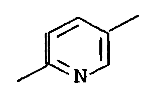 | CH₂Ph | CONH₂ |
| 45 | 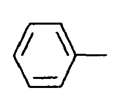 | -SO₂NH- | 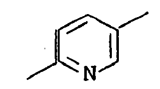 | CH₂Ph | H |
| 46 | 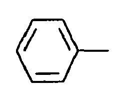 | -SO₂NH- | 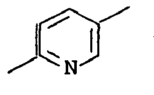 | CH₂Ph | CONH₂ |
| 47 | 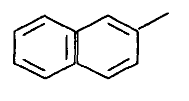 | -CONH- | 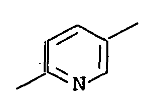 | CH₂Ph | H |
| 48 | 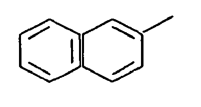 | -CONH- | 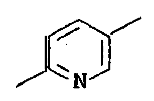 | CH₂Ph | CONH₂ |
| 49 | 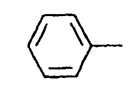 | ≡ | 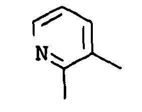 | CH₂Ph | H |
| 50 | 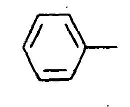 | ≡ | 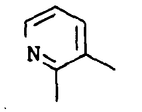 | CH₂Ph | CONH₂ |
| 54 | 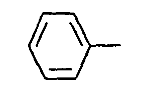 | -NHCO- | 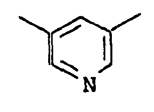 | CH₂Ph | H |
| 55 | 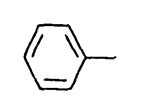 | -NHCO- | 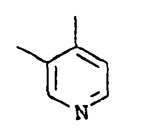 | CH₂Ph | CONH₂ |
| 56 | 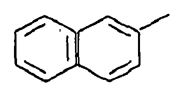 | -NHCO- | 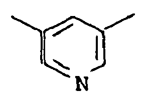 | CH₂Ph | CONH₂ |
| 57 | 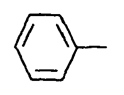 | 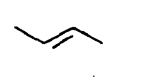 | 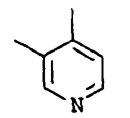 | CH₂Ph | H |
| 58 | 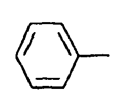 | 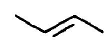 | 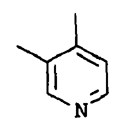 | CH₂Ph | CONH₂ |
| 59 | 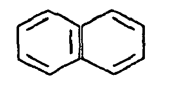 | 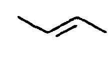 | 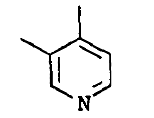 | CH₂Ph | CONH₂ |
| 60 | 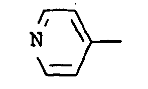 | 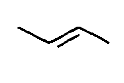 | 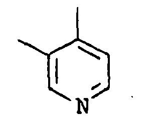 | CH₂Ph | CONH₂ |
| 61 | 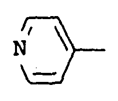 | 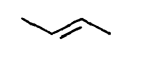 | 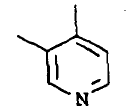 | CH₂Ph | 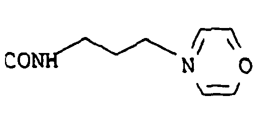 |
| 62 | 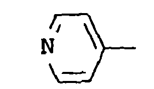 | 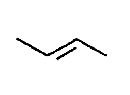 | 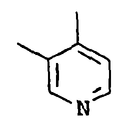 | CH₂Ph | 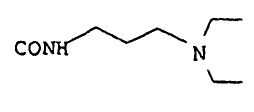 |
| 63 | 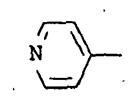 | 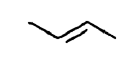 | 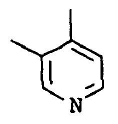 | CH₂Ph | 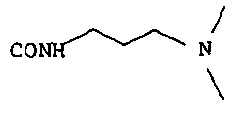 |
| 64 | 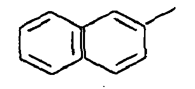 | -SO₂NH- | 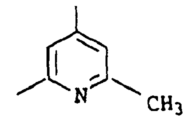 | CH₂Ph | CONH₂ |
| 65 | 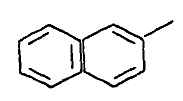 | -SO₂NH- | 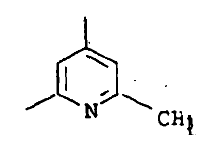 | CH₂Ph | H |
| 66 | 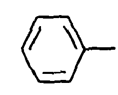 | -CH₂O- | 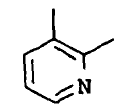 | CH₂Ph | H |
| 67 | 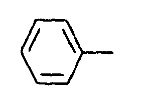 | -CH₂O- | 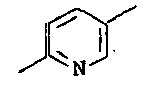 | CH₂Ph | CONH₂ |
| 68 | 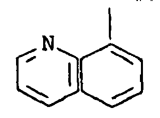 | -SO₂NH- | 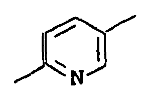 | CH₂Ph | CONH₂ |
| 69 | 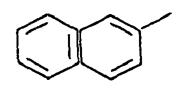 | -NHCO- | 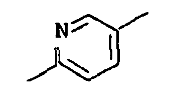 | CH₂Ph | H |
| 70 | 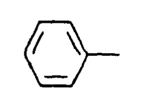 | -CH₂NHCO- | 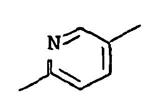 | CH₂Ph | CONH₂ |
| 71 | 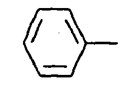 | -SO₂NH- | 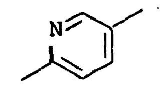 | CH₂Ph | |
| 72 | 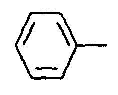 | -SO₂NH- | 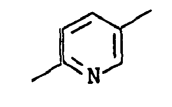 | CH₂Ph | 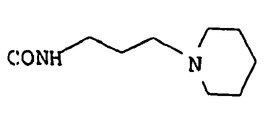 |
| 73 | 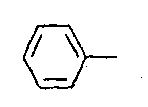 | SO₂NH - | 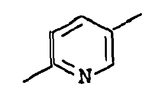 | CH₂Ph | 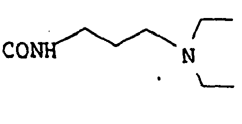 |
| 74 | 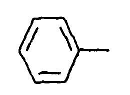 | -SO₂NH- | 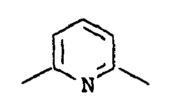 | CH₂Ph | CONH₂ |
| 75 | 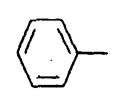 | -SO₂NH- | 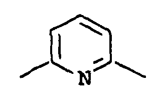 | CH₂Ph | 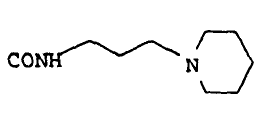 |
| 76 | 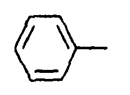 | -SO₂NH- | 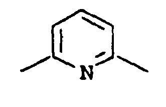 | CH₂Ph | 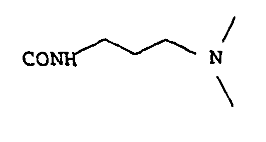 |
| 77 | 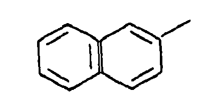 | -SO₂NH- | 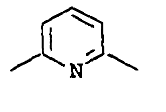 | CH₂Ph | CONH₂ |
| 78 | 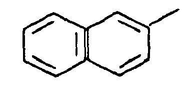 | -CH₂CH₂ | 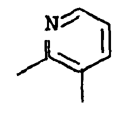 | CH₂Ph | CONH₂ |
| 79 | 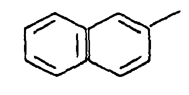 | -CH₂CH₂ | 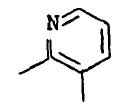 | CH₂Ph | H |
| 80 | 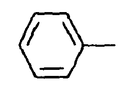 | 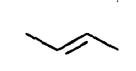 | 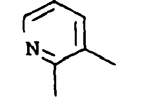 | 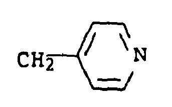 | 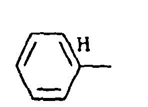 |
| 81 | 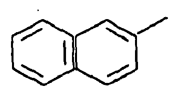 | 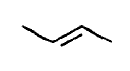 | 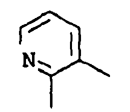 | 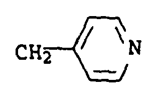 | 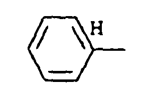 |

### Beispiel 86

### 2(4,6-dimethoxypyrimidin-1-yl)oxy-N(3-phenyl-propan-1-al-2-yl)-chinolin-4-carbonsäureamid

MS : m/e = 458 (M+)

### Beispiel 87

### N(3-Phenyl-propan-1-al-2-yl)-2-(2-pyridyl)oxy-8-trifluormethychinolin-4-carbonsäureamid

¹H-NMR (D₆-DMSO): δ = 3.0(1H), 3.4(1H), 4.9(1H), 7.3-8.9(13 H). 9.5(1H) und 9.9(1H) ppm.

### Beispiel 88

### N(3-Phenyl-propan-1-al-2-yl)-2(naphtho[c]pyrimidion-3-yl) -5-nikotinsäureamid

¹H-NMR (CF₃COOD): δ = 3.1-3.4(2H), 4.8(1H), 6.7(1H), 7.1-8.3(12 H), 8.7(1H) und 8.9(1H) ppm.

### Beispiel 89

### N(3-Chlorphenyl)carbamoyl-6-methyl-N(3-phenyl-propan-1-al-2-yl)-pyridin-3-carbonsäureamid

¹H-NMR (CF₃COOD): δ = 2.0-2.7(2H), 2.95(1H), 3.3-4.0(6H), 5.9(1 H), 6.9(1H), 7.0-7.5(10H) und 7.9(1H) ppm.

## Patentansprüche

1. Amide der allgemeinen Formel I 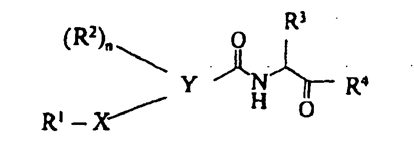 und ihre tautomeren Formen, möglichen enantiomeren und diastereomeren Formen, sowie mögliche physiologisch verträgliche Salze, worin die Variablen folgende Bedeutung haben:
R¹ Phenyl, Naphthyl, Chinolyl, Pyridyl, Pyrimidyl, Pyrazyl, Pyridazyl, Imidazolyl, Thiazol, Chinazyl, Isochinolyl, Chinoxalyl, Thienyl, Benzothienyl, Benzofuranyl, Furanyl, und Indolyl bedeuten kann, wobei die Ringe noch mit zu bis 3 Resten R⁵ substituiert sein können,
R² Chlor, Brom. Fluor, C₁ - C₆ - Alkyl, C₁ - C₆ - Alkenyl, C₁ - C₆ -Alkinyl, C₁ - C₆-Alkyl-Phenyl, C₁ - C₆-Alkenyl-Phenyl, C₁ - C₆-Alkinyl-Phenyl, Phenyl, NHCO-C₁ -C₄-Alkyl, NHSO₂-C₁-C₄-Alkyl, -NHCOPhenyl, -NHCO-Naphthyl, NO₂,-O-C₁-C₄-Alkyl und NH₂ bedeutet, wobei die aromatischen Ringe noch ein oder zwei Reste R⁵ tragen können, und zwei Reste R² können zusammen auch eine Kette -CH=CH-CN=CH- darstellen und somit einen anellierten Benzo-Ring bilden, der seinerseits mit einem R⁵ substituiert sein kann und
R³ -C₁-C₆-Alkyl, verzweigt oder unverzweigt, und der noch einen S-CH₃-Rest, Phenyl-, Cyclohexyl-, Cycloheptyl-, Cyclopentyl-, Indolyl-, Pyridyl- oder Naphthyl-Ring tragen kann, der seinerseits mit maximal zwei Resten R⁵ substituiert ist, wobei R⁵ Wasserstoff, C₁-C₄-Alkyl, verzweigt oder unverzweigt, -O-C₁-C₄-Alkyl, OH, Cl, F, Br, J, CF₃, NO₂ , NH₂, CN , COOH , COO-C₁-C₄-Alkyl, -NHCO-C₁-C₄-Alkyl, -NHCO-Phenyl, -NHSO₂-C₁-C₄-Alkyl, -NNSO₂-Phenyl, -SO₂-C₁-C₄-Alkyl, -(CH₂)ₙ-NR¹²R¹³ und -SO₂ Phenyl bedeutet,
X eine Bindung, -(CH₂)ₘ-, -(CH₂)ₘ-O-(CH₂)ₒ-, -(Ch₂)ₒ-S-(CH₂)ₘ-, -(CH₂)ₒ-SO-(CH₂)ₘ-, -(CH₂)ₒ-SO₂-(CH₂)ₘ-, -CH=CH-, -C≡C-, -CO-CH=CH-, -(CH₂)ₒ-CO-(CH₂)ₘ-, -(CH₂)ₘ-NHCO-(CH₂)ₒ-, -(CH₂)ₘ-CONH-(CH₂)ₒ-, -(CH₂)ₘ-NHSO₂(CH₂)ₒ-, -NH-CO-CH=CH-, -(CH₂)ₘ-SO₂NH-(CH₂)ₒ-, -CH=CH-CONH- und 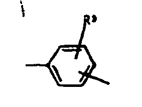 bedeutet,
und im Falle von CH=CH- Doppelbindungen sowohl die E als auch die Z-Form sein kann und
R¹-X zusammen auch 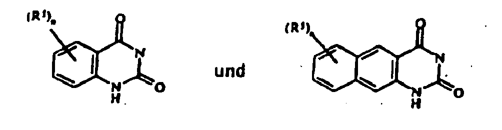 bedeuten und
Y Pyridin,
_{R}4 Wasserstoff, COOR⁶ und CO-Z bedeutet, worin Z NR⁷R⁸, und 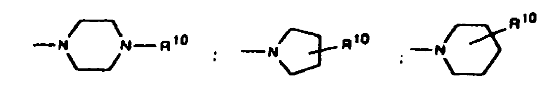 bedeutet,
R⁶ Wasserstoff, C₁-C₆-Alkyl, geradlinig oder verzweigt, bedeutet und das mit einem Phenylring substituiert kann, der selbst noch mit einem oder zwei Resten R⁹ substituiert sein kann, und
R⁷ Wasserstoff, C₁-C₆-Alkyl, verzweigt und unverzweigt, bedeutet, und
R⁸ Wasserstoff, C₁-C₆-Alkyl, verzweigt oder unverzweigt, das noch mit einem Phenylring, der noch einen Rest R⁹ tragen kann, und mit. 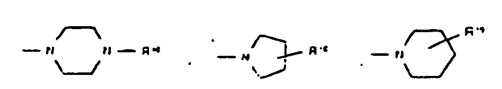 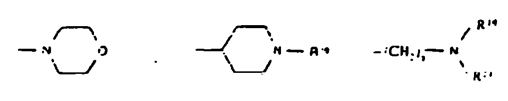 substituiert sein kann bedeutet, und
R⁹ Wasserstoff, C₁-C₄-Alkyl, verzweigt oder unverzweigt, -O-C₁-C₄-Alkyl, OH, Cl, F, Br, J, CF₃, NO₂ , NH₂ , CN , COOH, COO-C₁-C₄-Alkyl, -NHCO-C₁-C₄-Alkyl, -NHCO-Phenyl, -NHSO₂-C₁-C₄-Alkyl, -NHSO₂-Phenyl, -SO₂-C₁-C₄-Alkyl und-SO₂-Phenyl bedeuten kann
R¹⁰ Wasserstoff, C₁-C₆-Alkyl, geradlinig oder verzweigt, bedeutet und das mit einem Phenylring substituiert kann, der selbst noch mit einem oder zwei Resten R⁹ substituiert sein kann, und
R¹¹ Wasserstoff. C₁-C₆-Alkyl, geradlinig oder verzweigt, bedeutet und das mit einem Phenylring substituiert kann, der selbst noch mit einem oder zwei Resten R⁹ substituiert sein kann, und
n eine Zahl 0, 1 oder 2 bedeutet, und
m,o unabhängig voneinander eine Zahl 0, 1, 2, 3 oder 4 bedeutet.

2. Amide der Formel I gemäß Anspruch 1, wobei
R³ Benzyl, CH₂CH₂CH₂CH₃, CH₂CH₂CH₂CH₂CH₃ und
Y Pyridin und
R⁴ CO-NR⁷R⁸ und
R⁷ Wasserstoff
R⁸ CH₂CH_{2,} CH₂CH₂CH_{2,} CH₂CH₂CH₂CH₂ und
R⁹ Wasserstoff und
N 0 und 1 und
alle restlichen Variablen die gleiche Bedeutung wie im Anspruch 1 haben.

3. Amide der Formel I gemäß Anspruch 1, wobei
R³ Benzyl. CH₂CH₂CH₂CH₃, CH₂CH₂CH₂CH₂CH₃ und
Y Pyridin und
R⁴ Wasserstoff und
R⁹ Wasserstoff und
n 0 und 1 und
alle restlichen variablen die gleiche Bedeutung wie im Anspruch 1 haben.

4. Amide der Formel I gemäß einem der Ansprüche 1 - 3 als Arzneimittel.

5. Verwendung von Amiden der Formel I gemäß einem der Ansprüche 1 - 3 zur Herstellung von Arzneimitteln zur Behandlung von Krankheiten, bei denen erhöhte Calpain-Aktivitäten auftreten.

6. Verwendung von Amiden der Formel I gemäß einem der Ansprüche 1 - 3 zur Herstellung von Arzneimitteln zur Behandlung von Krankheiten, die auf die Inhibierung von Cysteinproteasen reagieren.

7. Verwendung nach Anspruch 6 zur Behandlung von Krankheiten, die auf die Inhibierung von Calpainen oder Cathepsinen reagieren.

8. Verwendung nach Anspruch 7 zur Behandlung von Krankheiten, die auf die Inhibierung der Cysteinproteasen Calpain I und II oder Cathepsin B und L reagieren.

9. Verwendung von Amiden der Formel I gemäß einem der Ansprüche 1 - 3 zur Herstellung von Arzneimitteln zur Behandlung von neuro-degenerativen Krankheiten und neuronalen Schädigungen.

10. Verwendung nach Anspruch 9 zur Behandlung von neuro-degenerativen Krankheiten und neuronalen Schädigungen, die durch Ischämie, Trauma oder Massenblutungen ausgelöst werden.

11. Verwendung nach Anspruch 9 zur Behandlung von Hirnschlag und Schädel-Hirntrauma.

12. Verwendung nach Anspruch 9 zur Behandlung der Alzheimerschen Krankheit und der Huntington-Krankheit.

13. Verwendung nach Anspruch 9 zur Behandlung von Epilepsien.

14. Verwendung der Verbindungen der Formel I gemäß einem der Ansprüche 1 - 3 zur Herstellung von Arzneimitteln zur Behandlung von Schädigungen des Herzens nach cardialen Ischämien, Reperfusionsschädigungen nach Gefäßverschlüssen, Schädigungen der Nieren nach renalen Ischämien, Skelettmuskelschädigungen, Muskeldystrophien, Schädigungen, die durch Proliferation der glatten Muskelzellen entstehen, coronarer Vasospasmus, cerebraler Vasospasmus, Katarakten der Augen und Restenosis der Blutbahnen nach Angioplastie.

15. Verwendung von Amiden der Formel I gemäß einem der Ansprüche 1 -3 zur Herstellung von Arzneimitteln zur Behandlung von Tumoren und deren Metastasierung.

16. Verwendung von Amiden der Formel I gemäß einem der Ansprüche 1 - 3 zur Herstellung von Arzneimitteln zur Behandlung von Krankheiten, bei denen erhöhte Interleukin-1-Spiegel auftreten.

17. Verwendung von Amiden der Formel I gemäß einem der Ansprüche 1-3 zur Herstellung von Arzneimitteln zur Behandlung von immunologischen Krankheiten.

18. Arzneimittelzubereitungen zur peroralen, parenteralen und intraperitonaien Anwendung, enthaltend pro Einzeldosis, neben den üblichen Arzneimittelhilfsstoffen, mindestens ein Amid 1 gemäß einem der Ansprüche 1 - 3.

## Claims

1. Amides of the general formula I 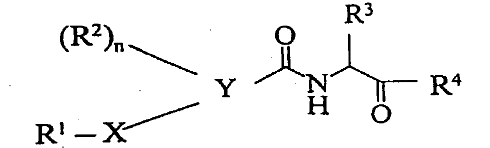 and their tautomeric forms, possible enantiomeric and diastereomeric forms, as well as possible
physiologically tolerable salts, in which the variables have the following meanings:
R¹ can be phenyl, naphthyl, quinolyl, pyridyl, pyrimidyl, pyrazyl, pyridazyl, imidazolyl, thiazole, quinazyl, isoquinolyl, quinoxalyl, thienyl, benzothienyl, benzofuranyl, furanyl, and indolyl, where the rings can be additionally substituted by up to 3 radicals R⁵,
R² is chlorine, bromine, fluorine, C₁-C₆-alkyl, C₁-C₆-alkenyl, C₁-C₆-alkynyl, C₁-C₆-alkylphenyl, C₁-C₆-alkenylphenyl, C₁-C₆-alkynylphenyl, phenyl, NHCO-C₁-C₄-alkyl, NHSO₂-C₁-C₄-alkyl, -NHCOphenyl, -NHCO-naphthyl, NO₂, -O-C₁-C₄-alkyl and NH₂, where the aromatic rings can additionally carry one or two radicals R⁵, and two radicals R² together can also be a chain -CH=CH-CH=CH- and thus form an anellated benzo ring, which for its part can be substituted by one R⁵, and
R³ is C₁-C₆-alkyl, which is branched or unbranched, and which can additionally carry an S-CH₃ radical or a phenyl, cyclohexyl, cycloheptyl, cyclopentyl, indolyl, pyridyl or naphthyl ring which for its part is substituted by at most two radicals R⁵, where R⁵ is hydrogen, C₁-C₄-alkyl, which is branched or unbranched, -O-C₁-C₄-alkyl, OH, Cl, F, Br, I, CF₃, NO₂, NH₂, CN, COOH, COO-C₁-C₄-alkyl, -NHCO-C₁-C₄-alkyl, -NHCO-phenyl, -NHSO₂-C₁-C₄-alkyl, -NHSO₂-phenyl, -SO₂-C₁-C₄-alkyl, -(CH₂)ₙ-NR¹²R¹³ and -SO₂-phenyl,
X is a bond, -(CH₂)ₘ-, -(CH₂)ₘ-O-(CH₂)ₒ-, -(CH₂)ₒ-S-(CH₂)ₘ-, -(CH₂)ₒ-SO-(CH₂)ₘ-, -(CH₂)ₒ-SO₂-(CH₂)ₘ-, -CH=CH-, -C≡C-, -CO-CH=CH-, -(CH₂)ₒ-CO-(CH₂)ₘ-, -(CH₂)ₘ-NHCO-(CH₂)ₒ-, -(CH₂)ₘ-CONH-(CH₂)ₒ-, -(CH₂)ₘ-NHSO₂-(CH₂)ₒ-, -NH-CO-CH=CH-, -(CH₂)ₘ-SO₂NH-(CH₂)ₒ-, -CH=CH-CONH- and 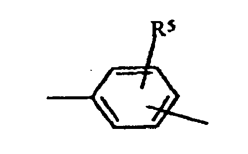 and in the case of CH=CH double bonds can be not only the E, but also the Z form, and
R¹-X together are also 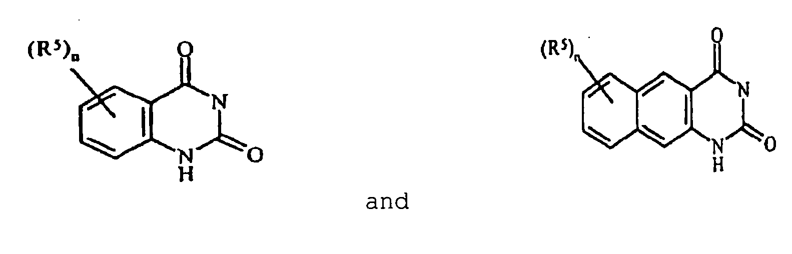 and
Y is pyridine,
R⁴ is hydrogen, COOR⁶, CO-Z, in which Z is NR⁷R⁸, and 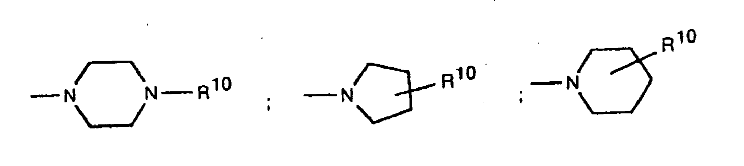
R⁶ is hydrogen, C₁-C₆-alkyl, which is linear or branched, and which can be substituted by a phenyl ring which itself can additionally be substituted by one or two radicals R⁹, and
R⁷ is hydrogen or C₁-C₆-alkyl, which is branched and unbranched, and
R⁸ is hydrogen, C₁-C₆-alkyl, which is branched or unbranched, which can additionally be substituted by a phenyl ring, which can additionally carry a radical R⁹, and by 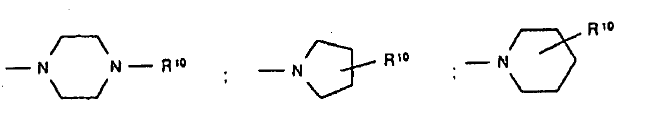 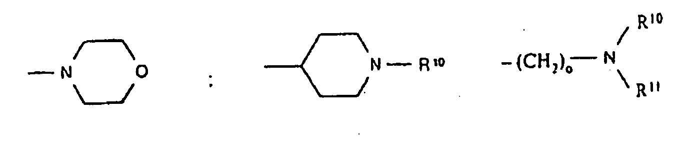 and
R⁹ can be hydrogen, C₁-C₄-alkyl, which is branched or unbranched, -O-C₁-C₄-alkyl, OH, Cl, F, Br, I, CF₃, NO₂, NH₂, CN, COOH, COO-C₁-C₄-alkyl, -NHCO-C₁-C₄-alkyl, -NHCO-phenyl, -NHSO₂-C₁-C₄-alkyl, -NHSO₂-phenyl, -SO₂-C₁-C₄-alkyl and -SO₂-phenyl,
R¹⁰ is hydrogen, C₁-C₆-alkyl, which is linear or branched, and which can be substituted by a phenyl ring which itself can additionally be substituted by one or two radicals R⁹, and
R¹¹ is hydrogen, C₁-C₆-alkyl, which is linear or branched, and which can be substituted by a phenyl ring which itself can additionally be substituted by one or two radicals R⁹, and
n is a number 0, 1 or 2, and
m, o independently of one another is a number 0, 1, 2, 3 or 4.

2. Amides of the formula I in accordance with claim 1, where
R³ is benzyl, CH₂CH₂CH₂CH₃, CH₂CH₂CH₂CH₂CH₃, and
Y is pyridine, and
R⁴ is CO-NR⁷R⁸, and
R⁷ is hydrogen,
R⁸ is CH₂CH₂, CH₂CH₂CH₂, CH₂CH₂CH₂CH₂, and
R⁹ is hydrogen, and
n is 0 and 1, and
all remaining variables have the same meanings as in claim 1.

3. Amides of the formula I in accordance with claim 1, where
R³ is benzyl, CH₂CH₂CH₂CH₃, CH₂CH₂CH₂CH₂CH₃, and
Y is pyridine, and
R⁴ is hydrogen, and
R⁹ is hydrogen, and
n is 0 and 1, and
all remaining variables have the same meanings as in claim 1.

4. Amides of the formula I in accordance with one of claims 1 - 3 as a medicament.

5. Use of amides of the formula I in accordance with one of claims 1 - 3 for the production of medicaments for the treatment of diseases in which increased calpain activities occur.

6. Use of amides of the formula I in accordance with one of claims 1 - 3 for the production of medicaments for the treatment of diseases that respond to the inhibition of cysteine proteases.

7. Use according to claim 6 for the treatment of diseases that respond to the inhibition of calpains or cathepsins.

8. Use according to claim 7 for the treatment of diseases that respond to the inhibition of cysteine proteases calpain I and II or cathepsin B and L.

9. Use of amides of the formula I in accordance with one of claims 1 - 3 for the production of medicaments for the treatment of neurodegenerative diseases and neuronal damage.

10. Use according to claim 9 for the treatment of neurodegenerative diseases and neuronal damage caused by ischemia, trauma or mass haemorrhages.

11. Use according to claim 9 for the treatment of cerebral stroke and craniocerebral trauma.

12. Use according to claim 9 for the treatment of Alzheimer's disease and Huntington's disease.

13. Use according to claim 9 for the treatment of epilepsy.

14. Use of the compounds of the formula I in accordance with one of claims 1 - 3 for the production of medicaments for the treatment of damage to the heart after cardiac ischemias, reperfusion damage after vascular occlusion, damage to the kidneys after renal ischemias, skeletal muscular damage, muscular dystrophies, damage which results due to proliferation of the smooth muscle cells, coronary vasospasm, cerebral vasospasm, cataracts of the eyes and restenosis of the blood vessels after angioplasty.

15. Use of amides of the formula I in accordance with one of claims 1 - 3 for the production of medicaments for the treatment of tumours and metastasis thereof.

16. Use of amides of the formula I in accordance with one of claims 1 - 3 for the production of medicaments for the treatment of diseases in which increased interleukin-1 levels occur.

17. Use of amides of the formula I in accordance with one of claims 1 - 3 for the production of medicaments for the treatment of immunological diseases.

18. Medicament preparations for peroral, parenteral and intraperitoneal administration, comprising per individual dose, in addition to the customary medicament auxiliaries, at least one amide I in accordance with one of claims 1 - 3.

## Revendications

1. Amides de la formule générale I 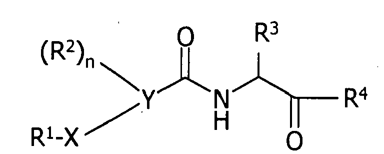 et leurs formes tautomères, formes énantiomères et diastéréomères possibles ainsi que sels physiologiquement compatibles possibles, dans laquelle les variables ont les significations suivantes :
R¹ peut être un groupe phényle, naphtyle, chinolyle, pyridyle, pyrimidyle, pyrazyle, pyridazyle, imidazolyle, thiazole, chinazyle, isochinolyle, chinoxalyle, thiényle, benzothiényle, benzofuranyle, furanyle et indolyle, les cycles pouvant encore être substitués avec jusqu'à 3 restes R⁵,
R² est le chlore, le brome, le fluor, un groupe alkyle en C₁-C₆, (alcényle en C₁-C₆, alcynyle en C₁-C₆, (alkyle en C₁-C₆ phényle, (alcényle en C₁-C₆ phényle, alcynyle en C₁-C₆ phényle, phényle, NHCO-(alkyle en C₁-C₄), NHSO₂-(alkyle en C₁-C₄), -NHCO-phényle, -NHCO-naphtyle, NO₂, -O-(alkyle en C₁-C₄) et NH₂, les cycles aromatiques pouvant encore porter un ou deux restes R⁵, et deux restes R² peuvent également représenter ensemble une chaîne -CH=CH-CH=CH- et former ainsi un cycle benzo condensé, lequel peut être pour sa part substitué avec un R⁵ et
R³ est un groupe alkyle en C₁-C₆, ramifié ou non ramifié, et lequel peut encore porter un reste S-CH₃, un cycle phényle, cyclohexyle, cycloheptyle, cyclopentyle, indolyle, pyridyle ou naphtyle, lequel est pour sa part substitué avec au maximum deux restes R⁵, R⁵ étant l'atome d'hydrogène, un groupe alkyle en C₁-C₄, ramifié ou non ramifié, -O-(alkyle en C₁-C₄), OH, CN, F, Br, J, CF₃, NO₂, NH₂, CI, COOH, COO-(alkyle en C₁-C₄),-NHCO-(alkyle en C₁-C₄), -NHCO-phényle, -NHSO₂-(alkyle en C₁-C₄), -NHSO₂-phényle, -SO₂-(alkyle en C₁-C₄), -(CH₂)ₙ-NR¹²R¹³ et -SO₂-phényle,
X est une liaison, -(CH₂)ₘ, -(CH₂)ₘ-O-(CH₂)ₒ-, -(CH₂)ₒ-S-(CH₂)ₘ-, -(CH₂)ₒ-SO-(CH₂)ₘ-, -(CH₂)ₒ-SO₂-(CH₂)ₘ-, -CH=CH-, -C≡C-, -CO-CH=CH-, -(CH₂)ₒ-CO-(CH₂)ₘ-, -(CH₂)ₘ-NHCO-(CH₂)ₒ-, -(CH₂)ₘ-CONH-(CH₂)ₒ-, -(CH₂)ₘ-NHSO₂-(CH₂)ₒ-, -NH-CO-CH=CH-, -(CH₂)ₘ-SO₂NH-(CH₂)ₒ-, -CH=CH-CONH- et 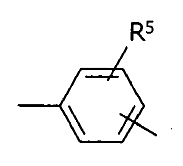 et peut être dans le cas de doubles liaisons CH=CH aussi bien la forme E que Z et
R¹-X sont également ensemble 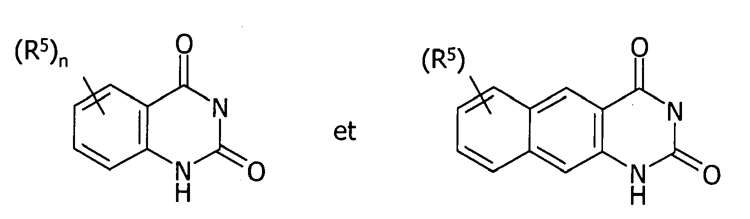 et
Y est le groupe pyridine,
R⁴ est l'atome d'hydrogène, COOR⁶ et CO-Z, où Z est NR⁷R⁸, et 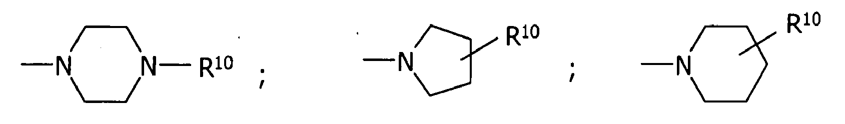
R⁶ est l'atome d'hydrogène, un groupe alkyle en C₁-C₆, linéaire ou ramifié, et lequel peut être substitué avec un cycle phényle, lequel peut encore être lui-même substitué avec un ou deux restes R⁹, et
R⁷ est l'atome d'hydrogène, un groupe alkyle en C₁-C₆ ramifié et non ramifié, et
R⁸ non peut est l'atome d'hydrogène, un groupe alkyle en C₁-C₆, ramifié ou ramifié, lequel peut encore être substitué avec un cycle phényle qui encore porter un reste R⁹, et avec 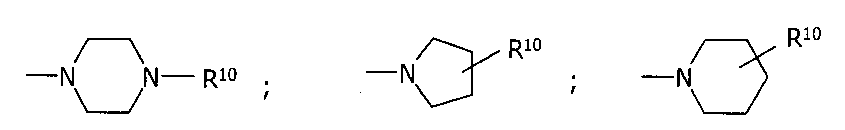 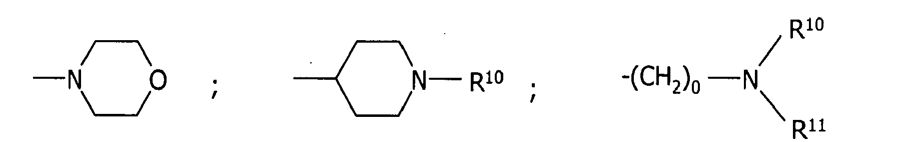
R⁹ est l'atome d'hydrogène, un groupe alkyle en C₁-C₄, ramifié ou non ramifié, -O-(alkyle en C₁-C₄), OH, CI, F, Br, J, CF₃, NO₂, NH₂, CN, COOH, COO-(alkyle en C₁-C₄), -NHCO-(alkyle en C₁-C₄), -NHCO-phényle, -NHSO₂-(alkyle en C₁-C₄), -NHSO₂-phényle, -SO₂-(alkyle en C₁-C₄) et -SO₂-phényle
R¹⁰ est l'atome d'hydrogène, un groupe alkyle en C₁-C₆, linéaire ou ramifié, et lequel peut être substitué avec un cycle phényle, lequel peut lui-même être encore substitué avec un ou deux restes R⁹, et
R¹¹ est l'atome d'hydrogène, un groupe alkyle en C₁-C₆, linéaire ou ramifié, et lequel peut être substitué avec un cycle phényle, lequel peut lui-même être encore substitué avec un ou deux restes R⁹, et
n représente un nombre de 0,1 ou 2, et
m, o représentent indépendamment un nombre de 0, 1, 2, 3 ou 4.

2. Amides de la formule I selon la revendication 1, dans laquelle
R³ est le groupe benzyle, CH₂CH₂CH₂CH₃, CH₂CH₂CH₂CH₂CH₃ et
Y est le groupe pyridine et
R⁴ est CO-NR⁷R⁸ et
R⁷ est l'atome d'hydrogène
R⁸ est CH₂CH₂, CH₂CH₂CH₂, CH₂CH₂CH₂CH₂ et
R⁹ est l'atome d'hydrogène et
n est égal à 0 et à 1 et
toutes les autres variables ont la même signification que dans la revendication 1.

3. Amides de la formule I selon la revendication 1, dans laquelle
R³ est le groupe benzyle, CH₂CH₂CH₂CH₃, CH₂CH₂CH₂CH₂CH₃ et
Y est le groupe pyridine et
R⁴ est l'atome d'hydrogène et
R⁹ est l'atome d'hydrogène et
n est égal à 0 et à 1 et
toutes les autres variables ont la même signification que dans la revendication 1.

4. Amides de la formule I selon l'une des revendications 1-3 comme médicament.

5. Utilisation d'amides de la formule I selon l'une des revendications 1-3 pour la préparation de médicaments destinés au traitement de maladies dans lesquelles apparaissent des activités élevées de calpaïne.

6. Utilisation d'amides de la formule I selon l'une des revendications 1-3 pour la préparation de médicaments destinés au traitement de maladies qui réagissent sur l'inhibition des cystéine protéases.

7. Utilisation selon la revendication 6 pour le traitement de maladies qui réagissent sur l'inhibition de calpaïnes ou de cathépsines.

8. Utilisation selon la revendication 7 pour le traitement de maladies qui réagissent sur l'inhibition des cystéine protéases calpaïne I et II ou cathépsine B et L.

9. Utilisation d'amides de la formule I selon l'une des revendications 1-3 pour la préparation de médicaments destinés au traitement de maladies neurodégénératives et de détériorations neuronales.

10. Utilisation selon la revendication 9 destinée au traitement de maladies neurodégénératives et de détériorations neuronales qui ont été provoquées par des ischémies, des traumatismes ou des hémorragies.

11. Utilisation selon la revendication 9 destinée au traitement d'attaques cérébrales et de traumatismes crâniens.

12. Utilisation selon la revendication 9 destinée au traitement de la maladie d'Alzheimer et de la maladie de Huntington.

13. Utilisation selon la revendication 9 destinée au traitement d'épilepsies.

14. Utilisation des composés de la formule I selon l'une quelconque des revendications 1-3 pour la préparation de médicaments destinés au traitement de détériorations du coeur après des ischémies cardiaques, de détériorations de ré-irrigation sanguine après des obstructions vasculaires, de détériorations des reins après des ischémies rénales, de détériorations des muscles du squelette, de dystrophies musculaires, de détériorations qui sont produites par prolifération des cellules lisses musculaires, d'angiospasmes coronaires, d'angiospasmes cérébraux, de cataractes des yeux et de resténoses des artères après une angioplastie.

15. Utilisation d'amides de la formule I selon l'une quelconque des revendications 1-3 pour la préparation de médicaments destinés au traitement de tumeurs et de leurs métastases.

16. Utilisation d'amides de la formule I selon l'une quelconque des revendications 1-3 pour la préparation de médicaments destinés au traitement de maladies dans lesquelles apparaît un niveau élevé d'interleukine-1.

17. Utilisation d'amides de la formule I selon l'une quelconque des revendications 1-3 pour la préparation de médicaments destinés au traitement de maladies immunologiques.

18. Préparations médicamenteuses destinées à une application perorale, parentérale et intrapéritonéale contenant par dose unitaire, en plus des auxiliaires classiques de médicaments, au moins un amide I selon l'une quelconque des revendications 1-3.
